(19) Europäisches Patentamt
European Patent Office
Office européen des brevets

(11) Publication number : **0 542 497 A1**

# (12) EUROPEAN PATENT APPLICATION

(21) Application number : **92310232.1**

(51) Int. Cl.[5] : **C07D 487/04,** A61K 31/505

(22) Date of filing : **09.11.92**

(30) Priority : **11.11.91 GB 9123916**

(43) Date of publication of application :
**19.05.93 Bulletin 93/20**

(84) Designated Contracting States :
**AT BE CH DE DK ES FR GB GR IE IT LI LU MC NL PT SE**

(71) Applicant : **THE WELLCOME FOUNDATION LIMITED**
**Unicorn House 160 Euston Road**
**London NW1 2BP (GB)**

(72) Inventor : **Kuyper, Lee Frederick**
**6 Plowlan Court**
**Durham, North Carolina 27707 (US)**
Inventor : **Jones, Michael Lee**
**5724 Roslyn Road**
**Durham, North Carolina 27712 (US)**
Inventor : **Baccanari, David Patrick**
**1415 Debra Drive**
**Cary, North Carolina 27511 (US)**

(74) Representative : **Rollins, Anthony John et al**
**Group Patents & Agreements The Wellcome Foundation Ltd Langley Court**
**Beckenham Kent BR3 3BS (GB)**

## (54) Heterocyclic compounds.

(57) Compounds of the formula (II) :

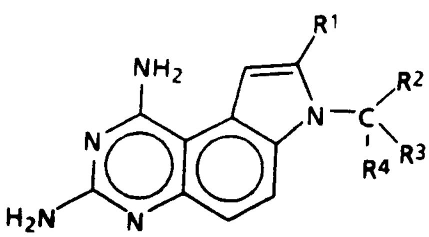

(II)

or acid addition salts thereof, wherein $R^1$ is hydrogen, $C_{1-6}$ alkyl or $C_{1-4}$ alkyl substituted by halo or $C_{1-4}$ alkoxy, $R^2$ is $C_{1-4}$ alkyl or $C_{1-4}$ alkyl substituted b halo or $C_{1-4}$ alkoxy, $R^3$ is $C_{1-4}$ alkyl or $C_{1-4}$ alkyl substituted by halo or $C_{1-4}$ alkoxy or

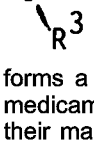

forms a $C_{5-7}$ cycloalkyl or cycloalkenyl group and $R^4$ is hydrogen or $C_{1-4}$ alkyl are described as useful medicaments in the treatment of cancer. Pharmaceutical compositions containing the compounds and their manufacture are also disclosed.

EP 0 542 497 A1

Jouve, 18, rue Saint-Denis, 75001 PARIS

The present invention relates to pyrroloquinazoline dihydrofolate reductase inhibitors, to methods for preparing them, to pharmaceutical compositions containing them and to their use in medicine.

US Patent No. 4118561 discloses inter alia compounds of the formula (I):

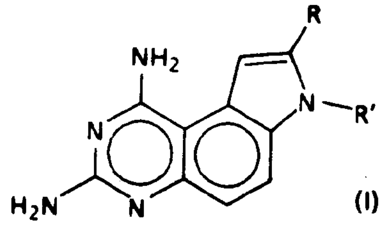

(I)

wherein X is hydrogen and Y is a group $CH_2R$ wherein R is hydrogen or a $C_{1-6}$ alkyl or $C_{4-6}$ cycloalkyl group or an optionally substituted phenyl group or Y is an optionally substituted aryl group, or X is methyl and Y is hydrogen, methyl or an optionally substituted benzyl group. These compounds are described as having antibacterial, antimalarial and antitumour activity, and as being inhibitors of the dihydrofolate reductase enzyme.

Methotrexate (N-[4[[(2,4-diamino-6-pteridinyl)methyl]methylamino]benzoyl]-L-glutamic acid), piritrexim(2,4-diamino-6(2,5-dimethoxybenzyl)5-methylpyrido(2,3-d)pyrimidine) and trimetrexate (5-methyl-6-[[(3,4,5-trimethoxyphenyl)amino]methyl]-2,4-quinazolinediamine) are all inhibitors of human dihydrofolate reductase which have demonstrated antitumour activity in the clinic. However, these compounds do not readily cross the blood/brain barrier and are consequently of limited utility against brain tumours. Trimethoprim(2,4-diamino-5-(3,4,5-trimethoxybenzyl)pyrimidine) is an inhibitor of bacterial dihydrofolate reductase which has been extensively used for the treatment of bacterial infections in humans.

It has now been discovered that a novel class of pyrroloquinazolines are capable of inhibiting tumour growth in mouse and mammalian cell lines at concentration levels equal or less than those required for methotrexate and piritrexim. In addition, these novel compounds are capable of crossing the blood/brain barrier. Accordingly, the present invention provides a compound of the formula (II):

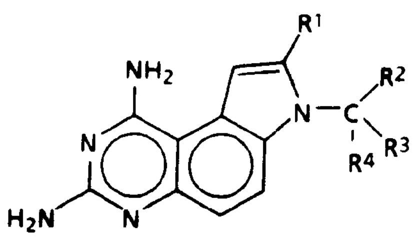

(II)

or an acid addition salt thereof, wherein $R^1$ is hydrogen, $C_{1-6}$ alkyl or $C_{1-4}$ alkyl substituted by halo or $C_{1-4}$ alkoxy, $R^2$ is a $C_{1-4}$ alkyl or $C_{1-4}$ alkyl substituted by halo or $C_{1-4}$ alkoxy, $R^3$ is a $C_{1-4}$ alkyl or $C_{1-4}$ alkyl substituted by halo or $C_{1-4}$ alkoxy or $C_{R_3}^{R2}$ forms a $C_{5-7}$cycloalkyl or cycloalkenyl group and $R^4$ is hydrogen or $C_{1-4}$ alkyl.

Suitably $R^1$ is hydrogen, $C_{1-4}$ alkyl or $C_{1-4}$ alkyl substituted by halo. Most suitably $R^1$ is hydrogen, methyl, ethyl, i-propyl, sec-butyl, t-butyl. In one preferred embodiment $R^1$ is methyl. In a second preferred embodiment $R^1$ is ethyl, i-propyl, sec-butyl or t-butyl.

Suitably $R^2$ is ethyl, propyl, butyl, or methoxymethyl. Preferably $R^2$ is ethyl or n-propyl.

Suitably $R^3$ is ethyl, propyl, butyl or methoxymethyl. Preferably $R^3$ is ethyl.

Suitably $R^4$ is hydrogen, methyl or ethyl. Preferably $R^4$ is hydrogen or methyl.

A preferred group of compounds of the formula (II) is that of the formula (IIa)

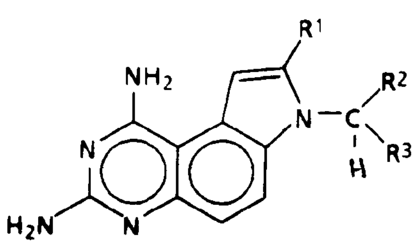

(IIa)

or an acid additon salt thereof, wherein $R^{1a}$ is hydrogen or $C_{1-4}$ alkyl, $R^{2a}$ is $C_{1-4}$ alkyl, and $R^{3a}$ is $C_{1-4}$ alkyl.

Suitably $R^{1a}$ is hydrogen, methyl or ethyl. Preferably $R^{1a}$ is hydrogen or methyl.

Suitably $R^{2a}$ is ethyl, n-propyl or n-butyl. Preferably $R^{2a}$ is ethyl or n-propyl.

Suitably $R^{3a}$ is ethyl, n-propyl or n-butyl. Preferably $R^{3a}$ is ethyl.

By "halo" is meant fluoro, chloro, bromo or iodo. Preferably halo represents fluoro or chloro.

By "alkyl" is meant straight or branched chain alkyl groups.

Salts of the compounds of the present invention comprise acid addition salts formed by protonation of a ring nitrogen in a compound of formula (II). The therapeutic activity resides in the moiety derived from the compound of the invention as defined herein and the identity of the other component is of less importance although for therapeutic and prophylactic purposes it is, preferably, pharmaceutically acceptable to the patient. Examples of pharmaceutically acceptable acid addition salts include those derived from mineral acids, such as hydrochloric, hydrobromic, phosphoric, metaphosphoric, nitric and sulphuric acids, and organic acids, such as tartaric, acetic, trifluouroacetic, citric, malic, lactic, fumaric, benzoic, glycollic, gluconic, succinic and methanesulphonic and arylsulphonic, for example, p-toluenesulphonic, acids. The pharmaceutically acceptable salts together with the salts which are not thus acceptable have utility in the isolation and/or the purification of the compounds of the invention, and the pharmaceutically acceptable salts are obtained by techniques well known in the art.

Compounds of the formula (II) may be prepared as hydrates. Such hydrates are included within the scope of the term "compounds of the formula (II)" and hence within the present invention.

The compounds of the formula (II) may be prepared

(i) by the alkylation of 7H-pyrrolo[3,2-f] quinazoline-1,3-diamine or the 8-alkyl derivative thereof, with the appropriate reagent $ZCHR^2R^3$, wherein $R^2$ and $R^3$ are as hereinbefore defined and Z is a leaving group, in the presence of a strong base.

(ii) by the cyclisation of a compound of the formula (III).

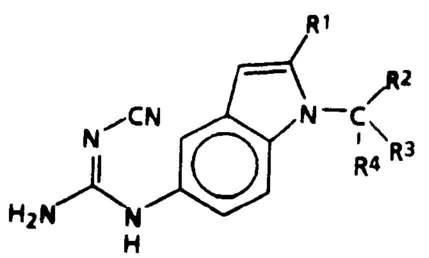

(III)

wherein $R^1$, $R^2$, $R^3$ and $R^4$ are as hereinbefore defined.

In reaction (i), Z is conveniently a halo atom, such as chloro, bromo or iodo, or a substituted sulphonyloxy group, such as methanesulphonyloxy or p-toluenesulphonyloxy group. The strong base must be capable of removing the proton from the indolic nitrogen and is conveniently sodium or potassium hydride, potassium t-butoxide or lithium or potassium amide. The reaction is conveniently carried out at an elevated temperature, for example 50°C to 150°C, in an inert solvent for example a dipolar aprotic solvent such as dimethylformamide or dimethylacetamide. 7H-Pyrrolo[3,2-f] quinozoline-1,3-diamine can conveniently be prepared as described in US Patent No. 4118561.

Reaction (ii) is conveniently carried out at a temperature of 150°C to 250°C, and preferably between 150°C and 200°C, in an aliphatic alcohol or ethereal solvent, preferably one having a boiling point in the range of the reaction temperature, for example diglyme, or in a pressure vessel if a lower boiling alcohol, e.g. methanol, is used to permit the desired reaction temperatures. Alternatively the cyclization reaction (ii) may be carried out at a temperature of 0°C to 100°C, and preferably between 25°C and 60°C in an ethereal solvent such as dimethoxyethane, diglyme, or tetrahydrofuran in the presence of a Lewis acid such as boron trifluoride. Compounds of the formula (III) may be prepared by a method known for analogous compounds, for example from compounds of the formula (IV) by the reaction of such a compound with dicyanamide.

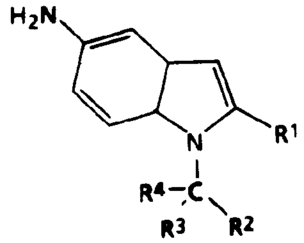

(IV)

Compounds of the formula (IV) may be prepared by methods known for analogous compounds, for example as shown in Schemes 1 and 2.

Whilst it is possible for the compounds or salts of the present invention to be administered as the raw chemical, it is preferred to present them in the form of a pharmaceutical formulation. Accordingly, the present invention further provides a pharmaceutical formulation, for medicinal application, which comprises a compound of the present invention or a pharmaceutically acceptable salt thereof, as hereinbefore defined, and a pharmaceutically acceptable carrier therefor.

The pharmaceutical formulation may optionally contain other therapeutic agents that may usefully be employed in conjunction with the compound or salt of the present invention, for example a pyrimidine nucleoside transport inhibitor that is capable of enhancing the antineoplastic activity of the compounds and salts of the present invention. The expression "pharmaceutically acceptable" as used herein in relation to the carrier is used in the sense of being compatible with the compound or salt of the invention employed in the formulation and with any other therapeutic agent that may be present, and not being detrimental to the recipient thereof. The carrier itself may constitute one or more excipients conventionally used in the art of pharmacy that enable the compound or salt of the present invention and any other therapeutic agent that may be present, to be formulated as a pharmaceutical formulation.

The pharmaceutical formulations of the present invention include those suitable for oral, parenteral (including subcutaneous, intradermal, intramuscular and intravenous) and rectal administration although the most suitable route will probably depend upon, for example, the condition and identity of the recipient. The formulations may conveniently be presented in unit dosage form and may be prepared by any of the methods well known in the art of pharmacy. All methods include the step of bringing into association the active ingredient, i.e., the compound or salt of the present invention, with the carrier. In general, the formulations are prepared by uniformly and intimately bringing into association the active ingredient with a liquid carrier or, a finely divided solid carrier or both, and then, if necessary, forming the associated mixture into the desired formulation.

The pharmaceutical formulations of the present invention suitable for oral administration may be presented as discrete units, such as a capsule, cachet, tablet, or lozenge, each containing a predetermined amount of the active ingredient; as a powder or granules; as a solution or a suspension in an aqueous liquid or a non-aqueous liquid such as a syrup, elixir or a draught, or as an oil-in-water liquid emulsion or a water-in-oil liquid emulsion. The formulation may also be a bolus, electuary or paste.

Generally, a tablet is the most convenient pharmaceutical formulation suitable for oral administration. A tablet may be made by compressing or moulding the active ingredient with the pharmaceutically acceptable carrier. Compressed tablets may be prepared by compressing in a suitable machine the active ingredient in a free-flowing form, such as a powder or granules, in admixture with, for example, a binding agent, an inert diluent, a lubricating agent, a disintegrating agent and/or a surface active agent. Moulded tablets may be prepared by moulding in a suitable machine a mixture of the powdered active ingredient moistened with an inert liquid diluent. The tablets may optionally be coated or scored and may be formulated so as to provide slow or controlled release of the active ingredient.

The pharmaceutical formulations of the present invention suitable for parenteral administration include aqueous and non-aqueous sterile injection solutions which may contain, for example, an anti-oxidant, a buffer, a bacteriostat and a solution which renders the composition isotonic with the blood of the recipient, and aqueous and non-aqueous sterile suspensions which may contain, for example, a suspending agent and a thickening agent. The formulations may be presented in uni-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions may be prepared from sterile powders, granules and tablets of the kind previously described.

The pharmaceutical formulations of the present invention suitable for rectal administration may be presented as a suppository containing, for example, cocoa butter and polyethylene glycol.

Compositions suitable for topical application to the skin preferably take the form of an ointment, cream, lotion, paste, gel, spray, aerosol, or oil. Carriers which may be used include vaseline, lanolin, polyethylene glycols, alcohols, and combinations of two or more thereof. The active compound of formula (II) is generally present at a concentration of from 0.1 to 10.0% w/w, for example, from 1.0 to 5.0% w/w.

Compositions for transdermal administration may be delivered by passive diffusion or by electrically assisted transport, for example, iontophoresis (see, for example, Pharmaceutical Research 3(6), 318, (1986)) and may take the form of an optionally buffered aqueous solution of a compound of formula (II). Typical compositions comprise citrate or bis/tris buffer (pH 6) or ethanol/water containing from 0.1 to 0.2M active ingredient, but other compositions suitable for administration by transdermal iontophoresis are within the scope of the present invention.

As mentioned hereinbefore, compounds and salts of formula (II) have anti-neoplastic activity as demonstrated hereinafter in the human colon adenocarcinoma HCT8 cell culture cytotoxicity tests in which representative compounds of the present invention have been shown to be active. Compounds and salts of formula (II) also have anti-neoplastic activity as determined hereinafter in the human breast MCF7 adenocarcinoma and human lymphoblastic leukemia (CCRF-CEM) cell culture cytotoxicity tests. It has thus been established that compounds of the present invention are able to inhibit neoplastic growth. Therefore, compounds and salts of the present invention are of use in medicine and in particular in the treatment of neoplastic growth, including solid tumours such as brain, lung, melanoma, breast and colon tumours in mammals. Accordingly, the present invention yet further provides a method for the treatment of susceptible malignant tumours and leukemia in an animal, e.g., a mammal, which comprises administering to the animal a therapeutically effective amount of a compound or salt of the present invention. In the alternative, there is also provided a compound or salt of the present invention for use in medicine and in particular for use in the treatment of a neoplastic growth, e.g., malignant tumours.

The compounds of the formula (II) have been found to partition into the CNS, for example the brain, rather than in plasma. Thus, it is believed that the compounds of the formula (II) may be of particular use in the treatment of tumours associated with the CNS, for example brain tumours and CNS leukemia.

The present invention also provides the use of a compound of formula (II) or a salt thereof for the manufacture of a medicament for the treatment of neoplastic growth.

The animal requiring treatment with a compound or salt of the present invention is usually a mammal, such as a human being.

Particular examples of a neoplastic growth requiring treatment include malignant tumours.

The compounds and salts of the present invention are believed to modify the function of the immune system of an animal to which they are administered. They may therefore be used to treat disorders of the immune system (e.g. rheumatoid arthritis and tissue rejection) and related diseases such as psoriasis. Accordingly, the present invention yet further provides a method for the treatment of disorders of the immune system especially rheumatoid arthritis, tissue rejection and related diseases such as psoriasis in an animal, e.g., a mammal, which comprises administering to the animal a therapeutically effective amount of a compound or salt of the present invention. In the alternative, there is also provided a compound or salt of the present invention for use in the treatment of disorders of the immune system, e.g. rheumatoid arthritis, tissue rejection and related diseases such as psoriasis.

The present invention also provides the use of a compound of formula (II) or a salt thereof for the manufacture of a medicament for the treatment of disorders of the immune system especially rheumatoid arthritis and tissue rejection and related diseases such as psoriasis.

The animal requiring treatment with a compound or salt of the present invention is usually a mammal such as a human being. Particular examples of disorders of the immune system requiring treatment include rheumatoid arthritis, tissue rejection and related diseases such as psoriasis.

Compounds and salts of the formula (II) also inhibit the enzyme Dihydrofolate Reductase from Escherichia coli, Pneumocystis carinii, Toxoplasma gondii and Candida albicans. Therefore, compounds and salts of the present invention may be of use in the treatment of bacterial (including mycobacterial), protozoal and fungal infections in mammals.

The route by which the compound or salt of the present invention is administered to the animal may be oral, topical, parenteral (including subcutaneous, intradermal, intramuscular, intravenous or rectal). If the compound or salt is presented in the form of a pharmaceutical formulation, which, as mentioned hereinbefore, is preferred, then the actual formulation employed will of course depend on the route of administration elected by the physician or veterinarian. For example, if oral administration is preferred, then the pharmaceutical formulation employed is, preferably, one which is suitable for such a route. Topical administration may be preferred in some instances, for example in the treatment of genital warts.

A therapeutically effective amount of a compound or salt of the present invention will depend upon a number of factors including, for example, the age and weight of the animal, the precise condition requiring treatment and its severity, the nature of the formulation, and the route of administration, and will ultimately be at the discretion of the attendant physician or veterinarian. However, an effective amount of a compound of the present invention for the treatment of bacterial, protozoal or fungal infections, or for the treatment of neoplastic growth, for example colon or breast carcinoma or for the treatment of genital warts will generally be in the range of 0.1 to 100 mg/kg body weight of recipient (mammal) per day and more usually in the range of 1 to 10 mg/kg body weight per day. Thus, for a 70kg adult mammal, the actual amount per day would usually be from 70 to 700 mg and this amount may be given in a single dose per day or more usually in a number (such as two, three, four, five or six) of sub-doses per day such that the total daily dose is the same. An effective amount of a salt of the present invention may be determined as a proportion of the effective amount of the compound per se.

An effective amount of a compound of the present invention for the treatment of disorders of the immune system (e.g. rhuematoid arthritis and tissue rejection) and related diseases such as psoriasis, will generally be in the range of 0.01-10 mg/kg body weight of recipient (mammal) per day. Thus for a 70 kg adult human, the actual amount per day would usually be from about 0.7-700 mg per day and this amount may be given in a single dose per day, or more usually dosing would be intermittent e.g. at 12 hourly intervals or weekly. An effective amount of a salt of the present invention may be determined as a proportion of the effective amount of the compound per se.

The treatment of bacterial, protozoal, fungal infections or, neoplastic growth with a compound of the present invention may at times require the administration to the animal of an antidote or rescue agent, e.g. leucovorin. This will be particularly preferred when treating tumours associated with the CNS, into which leucovorin will not partition to a substantial extent. The leucovorin may be administered with a compound of the present invention or prior or after such administration.

The following examples illustrate the preparation of compounds of the present invention and their pharmacological properties.

## Experimental Section

Melting points were determined with a Thomas-Hoover melting point apparatus and are uncorrected. Chromatography was carried out on E.Merck silica gel 60 (particle size 0.040-0.063 mm) using the solvents listed under the individual experiments. $^1H$ NMR spectra were measured at 200 MHz with a Varian XL-200 spectrometer. Chemical shifts are in parts per million (δ), relative to the observed solvent resonance (eg DMSO, 2.50). Multiplicities are designated as singlet (s), doublet (d), triplet (t), multiplet (m), and broad (br). Chemical ionization (CI) mass spectra were obtained by Oneida Research Services, Whitesboro, NY, using methane as the reagent gas. Elemental analyses were performed by Atlantic Microlab, Inc., Atlanta, GA.

## Abbreviations

MeOH = Methanol
EtOH = ethanol
DMSO = dimethylsulphoxide
EtOAc = ethylacetate
TLC = thin layer chromatography
HPLC = high performance liquid chromatography
$MgSO_4$ = magnesium sulfate
TEA = triethylamine
TFA = trifluoroacetic acid
$Et_3N$ = triethylamine
$CDCl_3$ = deuterated chloroform

## Example 1

### 1,3-Diamino-7-(1-ethylpropyl)-7H-pyrrolo(3,2-f)quinazoline

a) N-Cyano-N′(indol-5-yl)guanidine

A suspension of 8.3g 5-aminoindole hydrochloride (Aldrich), 11g sodium dicyanamide and 100ml dimethylformamide was heated at 40°C for 3 hours. The solvent was concentrated in vacuo and the remainder was

poured into 500ml water and stirred 2 hours. Vacuum filtration gave a solid which was washed with water (2x300ml) and dried in vacuo to give 8.9g solid, m.p. 238-239°C, NMR (DMSO-$d_6$): 6.4(bs, 1H), 6.7(bs, 2H), 6.95(m, 1H), 7.3(m, 2H), 7.45(S, 1H), 8.9(S,1H), 11.1(S, 1H). Anal. Calcd for: $C_{10}H_9N_5$: C, 60.03; H, 4.59; N, 35.06. Found: C, 60.21; H, 4.60; N, 35.10.

b) 1,3-Diamino-7H-pyrrolo(3,2-f)quinazoline monohydrate

Method A

A suspension of 166g N-cyano-N'(indol-5-yl)guanidine and 2.5 litres diglyme was refluxed 35 hours. The reaction was filtered, chilled and acidified with 143ml glacial acetic acid. Filtration provided 177g of the product as the diacetate salt. Anal.Calcd. for: C, 52.66; H, 5.37; N, 21.93. Found: C, 52.54; H, 5.45; N, 21.82. The salt was dissolved in 2.6 litres water and the pH adjusted to 12 with aqueous sodium hydroxide. Filtration gave 7H-pyrrolo(3.2-f)quinazoline-1,3-diamine monohydrate, m.p. 265-267°C. NMR (DMSO-$d_6$): 5.7(bs, 2H), 6.7(bs, 2H), 7.0(m, 2H), 7.4(m, 1H), 7.6(d, 1H), 11.5(bs, 1H).
Anal.Calcd. for $C_{10}H_9N_5.H_2O$: C, 55.29; H, 5.10; N, 32.24.
Found: C, 55.39; H, 5.14; N, 32.19.

Method B

A suspension of 1.0g N-cyano-N'(indol-5-yl)guanidine and 75ml methanol was placed in a Parr pressure reaction vessel and heated to 150°C for 35 hours (ca.300 p.s.i.). The mixture was cooled to room temperature, silica gel was added and the solvent was removed in vacuo to give a suspension on silica gel. Chromatography (silica gel, 20% ethanol in ethyl acetate with 0.5% ammonium hydroxide) afforded 0.49g of the expected product, which was stirred with 1.0 N sodium hydroxide and filtered to produce the free base.

c) 1-Ethylpropyl-4-toluenesulfonate

A suspension of 180g 3-pentyl alcohol, 1000ml methylene chloride, 600ml pyridine and 307g p-toluenesulfonyl chloride was stirred for 24 hours. A solution of ca. 300ml concentrated hydrochloric acid in 1000ml water was cautiously added to the reaction mixture. The mixture was stirred for 2 hours and the methylene chloride layer was separated. The water layer was extracted with diethyl ether and the extractions were combined with the methylene chloride layer. The volume of the organic solution was reduced to ca. 500ml and washed with 0.1 N hydrochloric acid until the aqueous layer remained acidic. The organic layer was then washed thoroughly with water, dried ($MgSO_4$), and filtered, and the solvent was removed in vacuo to give 230g white solid, m.p. 44-45°C. TLC (silica, 1:1 $CH_2Cl_2$/hexane): $R_f$.35. HPLC (Nova Pak $C^{18}$, 70% MeOH/$H_2O$/0.1% $Et_3N$/0.1% TFA): K'2.67 NMR ($CDCl_3$): 0.8(t, 6H), 1.6(m, 4H), 2.4(s, 3H), 4.45(m, 1H), 7.3(d, 2H), 7.8(d, 2H). Anal.Calcd. for: $C_{12}H_{18}O_3S$: C, 59.48; H, 7.49. Found: C, 59.44; H, 7.51.

d) 1,3-Diamino-7-(1-ethylpropyl)-7H-pyrrolo[3,2-f]quinazoline monohydrate

Method A

A suspension of 18.0g 1,3-diamino-7H-pyrrolo[3,2-f]quinazoline in 800ml dry dimethylformamide was stirred under nitrogen while 4.0g ca. 97% sodium hydride was added. The mixture was stirred 1 hour, and a solution of 23g 1-ethylpropyl-4-toluenesulfonate in ca. 80ml dry dimethylformamide was added dropwise during ca. 30 min. Stirring was continued for ca. 8 hours and the solvent was removed in vacuo. The dark residue was dried in vacuo and was dissolved in ca. 300ml boiling methanol. The solution was treated with charcoal and filtered through celite. The resulting dark solution was boiled down to ca. 200ml and allowed to cool. The resulting crystalline solid was isolated by filtration and washed with (2x50ml) cold methanol, sonicated with ca. 200ml 0.1 N sodium hydroxide and filtered. The solid was then washed (2x50ml) with water and dried in vacuo to afford 12.5g of the title compound, m.p. 197-198°C, NMR (DMSO-$d_6$): 0.64(t, 6H), 1.85(m, 4H), 4.32(m, 1H), 5.62(s, 2H), 6.62(bs, 2H), 7.02(d, 1H), 7.07(d, 1H), 7.51(d, 1H), 7.80(d, 1H) $\lambda_{max}$ (0.1 N HCl): 342($\varepsilon$ 11,208), 230($\varepsilon$ 26,874), 257 sh($\varepsilon$ 18,339)nm; $\lambda_{min}$: 284($\varepsilon$ 846), 219($\varepsilon$ 22,396)nm. HPLC (Nova Pak $C^{18}$, 30% MeOH/$H_2O$ with 0.1% TEA, 0.1% TFA) ret time 5.38 min. Anal.Calcd. for $C_{15}H_{19}N_5.H_2O$: C, 62.70; H, 7.37; N, 24.37. Found: C, 62.53; H, 7.36; N, 24.24.

Method B

A suspension of 18.0g 1,3-diamino-7H-pyrrolo[3,2-f]quinazoline monohydrate in 800ml dry dimethylformamide was stirred under nitrogen while 4.0g ca. 97% sodium hydride was added. The mixture was stirred for one hour, and a solution of 24g 1-ethylpropyl-4-toluenesulfo- nate in ca. 120ml dry dimethylformamide was added dropwise during ca. 30 min. Stirring was continued for ca. 8 hours and the solvent was removed in vacuo. The residue was dissolved in methanol and silica gel was added. The methanol was then removed in vacuo to suspend the residue on silica gel. Chromatography (silica gel, 10% ethanol in ethyl acetate) afforded the product which was sonicated in 1.0 N sodium hydroxide to yield the target compound as the free base.

Example 2

1,3-Diamino-7-(1-ethylpropyl)-8-methyl-7H-pyrrolo(3,2-f)quinazoline (Compound 2)

5-Nitro-2-methyl-1-(1-ethylpropyl)indole

Method A

(i) 2-Methyl-1-(1-ethylpropyl)indole

A suspension of 58g 2-methylindole in 2L dry dimethylformamide was stirred under nitrogen as 12.5g ca. 97% sodium hydride was carefully added. The mixture was stirred for one hour, and a solution of 1-ethylpropyl-4-toluenesulfonate in 350ml dry dimethylformamide was added in ca. 3 hours. The mixture was stirred overnight. The solvent was removed in vacuo and ca. 2L ethylacetate was added. The mixture was filtered and silica gel was added to the filtrate. Removal of the solvent in vacuo afforded the crude material suspended on silica gel. Chromatography (silica gel, hexane) gave 63g of the expected product. TLC (5% EtOAc/hexane, silica gel): $R_f$.39 NMR (DMSO-$d_6$): 0.63(t, 6H), 2.15(m, 4H), 2.38(s, 3H), 4.05(bs, 1H), 6.17(s, 1H), 6.93-7.4(m, 4H). HPLC (Nova Pak $C^{18}$, 80% MeOH/$H_2O$/0.1% $Et_3N$/0.1% TFA): K'4.02. Anal.Calcd. for $C_{14}H_{19}N$: C, 85.53; H, 9.51; N, 6.96. Found: C, 83.41; H, 9.53; N, 6.88.

(ii) 5-Nitro-2-methyl-1-(1-ethylpropyl)indole

A suspension of 31g 2-methyl-1-(1-ethylpropyl)indole in 200ml concentrated sulfuric acid was cooled to -10°C. A solution of 14g sodium nitrate in concentrated sulfuric acid was added dropwise in 2 hours while keeping the temperature below 0°C. The reaction was stirred 15 min. and then slowly poured into ca. 1.5L ice. The resulting yellow precipitate was collected by filtration and washed with water. Drying in vacuo provided 39g of the expected product. m.p. 69-71° TLC (silica gel, 4:1 hexane/ethyl acetate): Rf.48. HPLC (Nova Pak $C^{18}$, 80% MeOH/$H_2O$/0.1% $Et_3N$/0.1% TEA): K'2.56. NMR (DMSO-$d_6$): 1.6(6, 6H), 1.8-2.2(m, 4H), 2.4(s, 3H), 4.15(bs, 1H), 6.5(bs, 1H), 7.7(d, 1H), 7.9(m, 1H, 8.4(d, 1H). Anal.Calcd. $C_{14}H_{18}N_2O_2$: C, 68.27; H, 7.37; N, 11.37. Found: C, 68.33; H, 7.37; N, 11.41

Method B

(i) 2-(1-Propynyl)-N-(1-ethylpropyl)-4-nitroaniline

1-Propyne (2,7ml, 0.048mol) was condensed and added to a cold solution of 4.0g (.012mol) N-(1-ethylpropyl)-2-iodo-4-nitroaniline dissolved in 150ml triethylamine in a Parr Pressure Reaction vessel. To this solution was added 0.03g copper iodide and 0.1g bis(triphenylphosphine)palladium (II) chloride and the pressure vessel was then sealed. The mixture was stirred at room temperature for 20 hours and then removed from the pressure vessel. The solvent was removed in vacuo to leave a residue which was suspended on silica gel. The silica gel suspension of the residue was placed on a silica gel column and eluted with 10% ethyl acetate in hexane which provided 2,5g yellow oil. NMR (DMSO-$d_6$): 7.98(s, 1H), 7.90(s, 1H), 6.75(d, 1H), 5.8(d, 1H), 3.5(m, 1H), 2.1(s, 3H), 1.5(m, 4H), 0.8(t, 6H). Anal. Calcd for $C_{14}H_{18}N_2O_2$.0.2 $H_2O$: C, 67.28; H, 7.42; N, 11.21. Found: C, 67.34; H, 7.27; N, 11.23

(ii) 2-Methyl-1-(1-ethylpropyl)-5-nitro-1H-indole

A suspension of 2.5g 2-(1-propynyl)-N-(1-ethylpropyl)-4-nitro-aniline and 0.20g copper iodide in ca. 200ml

dimethylformamide was refluxed 5 hours and then allowed to cool to room temperature. The solvent was removed in vacuo to give a dark residue which was taken up in methanol, silica gel was added and the solvent removed in vacuo. The silica gel suspension of the residue was placed on a silica gel column and eluted with 5% ethyl acetate in hexane which gave 2.25g product as an oil, NMR (DMSO-$d_6$): 8.4(m, 1H), 7.9(br d, 1H), 7.7(d, 1H), 6.5(br s, 1H), 4.2(m, 1H), 1.8-2.2(m, 4H), 0.6(t, 6H). Anal. Calcd for $C_{14}H_{18}N_2O_2$: C, 68.27; H, 7.37; N, 11.37. Found: C, 68.03; H, 7.44; N, 11.27.

a) 5-Amino-2-methyl-1-(1-ethylpropyl)indole hydrochloride

A mixture of 2.5g 5-nitro-2-methyl-1-(1-ethylpropyl)indole, ca. 100ml methanol and 0.3g 10% palladium-charcoal was hydrogenated using a Parr hydrogenation apparatus (40 p.s.i.). After 2 hours, the mixture was filtered through celite and 3ml concentrated hydrochloric acid was added to the filtrate. The solution was evaporated in vacuo to dryness, whereupon absolute ethanol was added and evaporated in vacuo again. This sequence was repeated several times to remove excess hydrochloric acid. The final residue was vacuum-dried to give 2.5g of the hydrochloride salt of the expected product. HPLC (Nova Pak $C^{18}$, 60% $MeOH/H_2O$/0.1% $Et_3N$/0.1% TEA): K'2.50. NMR (DMSO-$d_6$): 1.4(t, 6H), 1.8-2.2(m, 4H), 2.4(s, 3H), 4.1(bs, 1H), 6.3(s, 1H), 6.9(d, 1H), 7.4(s, 1H), 7.6(d, 1H), 10.1(s, 3H). Anal.Calcd $C_{14}H_2ON_2$.HCl: C, 66.52; H, 8.37; N, 11.08; Cl, 14.02. Found: C, 66.28; H, 8.39; N, 11.04; Cl, 13.94.

b) N-Cyano-N'-2-methyl-1-((1-ethylpropyl)indol-5-yl)guanidine

A suspension of 2.4g 5-amino-2-methyl-1-(1-ethylpropyl)indole hydrochloride and 2.5g sodium dicyanamide in 125ml dimethylformamide was heated to 50°C for 4 hours. Solvent was removed in vacuo and water (ca. 250ml) was added. The mixture was stirred one hour and was filtered to give 2.8g product m.p. 151-152°C. TLC (3:1 EtOAc/hexane): $R_f$0.2. HPLC (Nova Pak $C^{18}$, 65% $MeOH/H_2O$/0.1% $Et_3N$/0.1% TFA): K'3.1 NMR (DMSO-$d_6$): 0.64(t, 6H), 1.79-2.19(m, 4H), 2.38(s, 3H), 4.05(m, 1H), 6.17(s, 1H), 6.83(s, 2H), 6.88-7.45(m, 3H), 8.95(s, 1H). Anal.Calcd $C_{16}H_{21}N_5$: C, 67.82; H, 7.47; N, 24.71. Found: C, 67.22; H, 7.50; N, 24.64.

c) 1,3-Diamino-7-(1-ethylpropyl)-8-methyl-7H-pyrrolo(3,2-f)quinazoline (Compound 2)

Method A

A suspension of 16g N-cyano-N'-(2-methyl-1-(1-ethylpropyl)indol-5-yl)guanidine in ca. 150ml diglyme was refluxed 40 hours. The mixture was cooled to room temperature, and 7ml glacial acetic acid and ca. 400ml diethyl ether was added. The mixture was stirred for 20 min. and filtered to give 11g solid. This solid was vigorously stirred with 175ml 1.0N sodium hydroxide for 3 hours and filtered. The solid was washed with water and vacuum-dried to give 9.9g of the expected product as a tan solid. m.p. 153-155°C. HPLC (Nova Pak $C^{18}$, 55% $MeOH/H_2O$/0.1% $Et_3N$/0.1% TFA): K'3.65 NMR (DMSO-$d_6$): 0.64(t, 6H), 1.82-2.38(m, 4H), 2.44(s, 3H), 4.11(m, 1H), 5.64(bs, 2H), 6.60(bs, 2H), 6.80(s, 1H), 6.92(d, J=8.8Hz, 1H, arom), 7.78(d, J=9.1Hz, 1H, arom). Anal.Calcd $C_{16}H_{21}N_5$.0.2$H_2O$: C, 66.97; H, 7.52; N, 24.40. Found: C, 66.96; H, 7.63; N, 24.28

Method B

A suspension of 2.6g N-cyano-N'-2-methyl-1-(1-ethylpropyl)indol-5-yl)guanidine in ca. 300ml methanol was placed in a Parr pressure reactor and heated to 150°C. After 35 hours, the reactor was cooled and silica gel was added to the methanol. The solvent was removed in vacuo to suspend the reaction material on silica gel. Chromatography (silica gel, 10% ethanol in methylene chloride) afforded a material which was sonicated with ca. 200ml 0.1 N sodium hydroxide for 1 hour and then filtered. Vacuum-drying gave 0.87g of the expected product m.p. 153-155°C, TLC (silica gel, 1:1 EtOH/EtOAc with 1% $NH_4OH$): Rf=0.51

Method C

A stirred suspension of 3.1g N-cyano-N'-(2-methyl-1-(1-ethylpropyl)indol-5-yl)guanidine in ca. 200ml dimethoxyethane was treated with 4.1ml (4.68g) boron trifluoride etherate. The resulting dark solution was stirred at room temperature 48 hours and concentrated in vacuo. The resulting solid was stirred with 250ml ammonium hydroxide for at least 3 hours and filtered. The solid was taken up in methanol, silica gel was added and the solvent removed in vacuo to provide a suspension on silica gel. The silica gel suspension of the residue was placed on a silica gel column and eluted with 10% ethanol in ethyl acetate. The resulting solid was dried

in vacuo (50°C, 2mm Hg) to give 0.6g of the product which exhibited NMR spectral properties identical to those of the product obtained by Method A.

d) 1,3-Diamino-7-(1-ethylpropyl)-8-methyl-7H-pyrrolo(3,2-f)quinazoline-methane sulfonate salt

A solution of 3.0g 1,3-diamino-7-(1-ethylpropyl)-8-methyl-7H-pyrrolo(3,2-f)-quinazoline in ca. 100ml methanol was added dropwise to a solution of 0.74ml methane sulfonic acid in ca. 10ml methanol. The mixture was stirred at 25°C for 1 hour and then the solvent was removed in vacuo to give a light brown residue. Diethyl ether (200ml) was added to the residue and sonicated for 1 hour. The mixture was then filtered to give 4.0g of a light brown solid. The solid was recrystallized twice from 5:1 diethyl ether/methanol and dried in vacuo (65°/mm Hg) to provide 2.85g of the expected methane sulfonate salt. NMR (DMSO-$d_6$): 12.2(s, 1H), 8.8(s, 1H), 8.1(d, 1H), 7.6(br s, 1H), 7.5(br s, 2H), 7.1(m, 2H), 4.2(m, 1H), 2.4(s, 3H), 1.8-2.2(m, 4H), 0.06(t, 6H). Anal. Calcd for $C_{17}H_{25}N_5SO_3.0.1H_2O$: C, 53.55; H, 6.66; N, 18.37; S, 8.41. Found: C, 53.60; H, 6.66; N, 18.35; S, 8.38.

Example 3

1,3-Diamino-7-(1-ethylbutyl)-7H-pyrrolo[3,2-f]quinazoline

a) 1-Ethylbutyl-4-toluenesulfonate

This compound was prepared according to the procedure for 1-ethylpropyl-4-toluenesulfonate in example 1 using 3-hexanol instead of 3-pentanol. A 94% yield of the title compound was obtained as an oil. NMR (DMSO-$d_6$): 0.71(t, 3H), 0.74(t, 3H), 1.15(m, 2H), 1.40-1.55(m, 4H), 2.40(s, 3H), 4.46(m, 1H), 7.45(d, 2H), 7.77(d, 2H). $\lambda_{max}$ (0.1N HC1): 222(ε 9600)nm. HPLC (Nova Pak $C^{18}$, 70% MeOH/$H_2O$ with 0.1% TEA, 0.1% TFA): K'=4.19 min.
Anal.Calcd $C_{13}H_{20}O_3S$: C, 60.91; H, 7.86; S, 12.51. Found: C, 61.09; H, 7.89; S, 12.35.

b) 1,3-Diamino-7-(1-ethylbutyl)-7H-pyrrolo[3,2-f]quinazoline

A suspension of 3.0g of 1,3-diamino-7H-pyrrolo[3,2-f]quinazoline in 150ml dry dimethylformamide was stirred under nitrogen while 0.4g ca. 97% sodium hydride was added. The suspension was stirred 1 hour and then chilled in an ice bath. A solution of 6.1g 1-ethylbutyl-4-toluenesulfonate in ca. 10ml dimethylformamide was added during ca. 45 min. The mixture was allowed to gradually warm to room temperature and stirred for ca. 8 hours. The solvent was removed (in vacuo) and the residue was crystallized from ca. 50ml methanol upon refrigeration overnight. The resulting crystals were stirred with ca. 25ml 0.1N sodium hydroxide, washed with water and dried to afford 0.4g of the title compound, m.p. 154-155°C, NMR (DMSO-$d_6$): 0.63(t, 3H), 0.77(t, 3H), 0.83-1.21(m, 2H), 1.74-1.90(m, 4H), 4.41(m, 1H), (br s, 2H, $NH_2$), 6.61 (br s, 2H, $NH_2$), 6.98-7.82(m, 4H, aromatic) $\lambda_{max}$ (0.1N HC1): 342(ε 25000), 259 sh (ε 16600)nm; $\lambda_{min}$; 287(ε 1000), 217(ε 21100)nm. HPLC (Nova Pak $C^{18}$, 70% MeOH/$H_2O$ with 0.1% TEA, 0.1% TFA): K'=0.69 min. Anal.Calcd $C_{16}H_{21}N_5.0.5H_2O$: C, 65.73; H, 7.58; N, 23.95. Found: C, 65.55; H, 7.42; N, 23.74.

Example 4

1,3-Diamino-7-(1-propylbutyl)-7H-pyrrolo[3,2-f]quinazoline hydrochloride(Compound 4)

a) 1-Propylbutyl-4-toluenesulfonate

This compound was prepared according to the procedure for 1-ethylpropyl-4-toluenesulfonate in example 1 using 4-heptanol instead of 3-pentanol. A 90% yield of the title compound was obtainted as an oil. NMR (DMSO-$d_6$): 0.74(t, 6H), 1.15(m, 4H), 1.46(m, 4H), 2.40(s, 3H), 4.50(m, 1H), 7.45(d, 2H), 7.77(d, 2H) $\lambda_{max}$ (0.1N HC1): 221(ε 7800)nm. HPLC (Nova Pak $C^{18}$, 70% MeOH/$H_2O$ with 0.1% TEA, 0.1% TFA): K'=6.95. Anal.Calcd $C_{14}H_{22}O_3S$: C, 62.19; H, 8.20; S, 11.86. Found: C, 62.29; H, 8.23; S, 11.79.

b) 1,3-Diamino-7-(1-propylbutyl)-7H-pyrrolo[3,2-f]quinazoline hydrochloride(Compound 4)

A suspension of 3.0g 7H-pyrrolo[3,2-f]quinazoline-1,3-diamine in 150ml dry dimethylformamide was stirred under nitrogen while 0.4g ca. 97% sodium hydride was added. After 1 hour, the mixture was chilled in an

ice bath and a solution of 6.5g 1-propylbutyl-4-toluenesulfonate in ca. 20ml dry dimethylformamide was added during ca. 45 min. The mixture was allowed to gradually warm to room temperature and stirred for ca. 8 hours. The solvent was removed (in vacuo) and the residue was taken up in methanol/methylene chloride. The solution was cooled to precipitate toluenesulfonic acid, which was removed by filtration. The filtrate was concentrated (in vacuo) to a gum and sonicated with 0.1N sodium hydroxide and water in succession. A portion of the gum was dissolved in 2-propanol and treated with 1N HC1 in diethyl ether giving the hydrochloride salt of title compound. m.p.>250°C, NMR (DMSO-$d_6$): 0.73-0.79(m, 6H), 0.85-1.16(m, 4H), 1.72-1.93(m, 4H), 4.60(m, 1H), 7.19-8.20(m, aromatic), 7.48(br s, 2H, $NH_2$), 8.80(br s, 1H), 12.7(br s, 1H, NH); $\lambda_{max}$(0.1N HC1): 350(ε 10,100), 228(ε 26,000), 260 sh(ε 17000)nm; $\lambda_{min}$: 286(ε 800), 217(ε 21,300). HPLC (Nova Pak $C^{18}$, 60% $MeOH/H_2O$ with 0.1% TFA, 0.1% TEA): K'=3.51. Anal.Calcd $C_{17}H_{23}N_5.0.5H_2O.1.00$ HC1: C, 59.55; H, 7.35; N, 20.43; Cl, 10.34. Found: C, 59.66; H, 7.32; N, 20.40; Cl, 10.42.

Example 5

1,3-Diamino-7-(tert-butyl)-8-ethyl-7H-pyrrolo(3,2-f)quinazoline (Compound 5)

a) N-tert-butyl-4-nitroaniline

A solution of 5.0g 4-fluoro-nitrobenzene (Aldrich), 7.8g tert-butylamine and 80ml dimethyl sulfoxide was heated to 50°C for 96 hours. The solvent was removed in vacuo to give a yellow oil which was dissolved in methanol. Silica gel was added and the solvent removed in vacuo. The silica gel suspension of the yellow oil was placed on a silica gel column and eluted with 10% ethyl acetate in hexane to afford the product which was dried in vacuo to give 5.7g yellow solid, m.p. 68-69°C, NMR (DMSO-$d_6$): δ 7.9(d, 2H), 7.0(br s, 1H), 6.7(d, 2H), 1.3(s, 9H). Anal. Calcd for $C_{10}H_{14}N_2O_2$: C, 61.84; H, 7.27; N, 14.42. Found: C, 61.77; H, 7.31; N, 14.33.

b) N-tert-butyl-2-iodo-4-nitroaniline

To a solution of 10g N-tert-butyl-4-nitroaniline in 500ml methanol was added 300ml of 1M iodine monochloride in methylene chloride (Aldrich) and the mixture was refluxed 3 hours. Aqueous sodium sulfite was slowly added with rapid stirring until the iodine colour discharged. The solution was extracted with ethyl acetate and the extract was washed with water and brine and evaporated in vacuo. The residue was taken up in methanol, silica gel was added and the solvent was removed in vacuo. The silica gel suspension of the residue was placed on a silica gel column and eluted with 20% ethyl acetate in hexane to provide 1.2g yellow solid, m.p. 115-116°C, NMR (DMSO-$d_6$): δ 8.5(d, 1H), 8.1(dd, 1H), 7.0(d, 1H), 5.2(br s, 1H), 1.4(s, 9H). Anal. Calcd for $C_{10}H_{13}N_2O_2I$: C, 37.52; H, 4.09; N, 8.75; I, 39.64. Found: C, 37.65; H, 4.07; N, 8.69; 1, 39.52.

c) N-tert-butyl-2-(1-butynyl)-4-nitroaniline

1-Butyne (10ml, 6.78g, 0.125mol) was condensed with a dry ice acetone condenser and added to a cold solution (-72°C) of 9.96g (.031mol) N-tert-butyl-2-iodo-4-nitroaniline dissolved in ca 400ml triethylamine in a Parr pressure reaction vessel. To this solution was added 0.06g copper iodide and 0.2g bis(triphenylphosphine) palladium (II) chloride and the pressure vessel sealed. The mixture was stirred at room temperature 20 hours after which the solvent was removed in vacuo. The residue was taken up in methanol, silica gel was added, and the solvent was removed in vacuo. The silica gel suspension of the residue was placed on a silica gel column and eluted with 5% ethyl acetate in hexane to give 6.8g solid product. m.p. 131-132°C, NMR (DMSO-$d_6$): δ 8.0(m, 2H), 7.0(d, 1H), 5.7(br s, 1H), 2.5(q, 2H), 1.2(t, 3H). Anal. Calcd for $C_{14}H_{18}N_2O_2$: C, 68.27; H, 7.37; N, 11.37. Found: C, 68.20; H, 7.40; N, 11.34.

d) 1-tert-butyl-2-ethyl-5-nitro-1H-indole

A suspension of 6.8g N-tert-butyl-2-(1-butynyl)-4-nitroaniline and 0.27g copper iodide in ca. 200ml anhydrous N,N-dimethylacetamide was heated to reflux ca 20 hours. The solvent was removed in vacuo, and the residue was taken up in methanol. Silica gel was added and the solvent was removed. The silica gel suspansion of the residue was placed on a silica gel column and eluted with 2% ethyl acetate in hexane to give 4.5g product, m.p. 121-123°C, NMR (DMSO-$d_6$): δ 8.4 (s, 1H), 7.9 (m,2H), 6.6 (s, 1H), 3.0 (q, 2H), 1.8 (s, 9H), 1.3 (t, 3H). Anal. Calcd for $C_{14}H_{18}N_2O_2$: C, 68.27; H, 7.37; N, 11.37. Found: C, 68.20; H, 7.40; N, 11.35.

e) 5-amino-2-ethyl-1-(tert-butyl)indole

This compound was prepared according to the method of example 2a.

A suspension of 4.5g N-tert-butyl-N'-ethyl-5-nitro-1H-indole in ca. 200ml methanol 200ml dimethylformamide and 0.20g 10% palladium on carbon was hydrogenated for 5 hours using a Parr hydrogenation apparatus (40 p.s.i.). The mixture was filtered through celite and the filtrate was evaporated in vacuo to remove the methanol which gave a solution of the expected product in dimethylformamide. This solution was used immediately to prepare N-cyano-N'-(2-ethyl-1-(tert-butyl)-1H-indol-5-yl)guanidine.

f) N-cyano-N'-(2-ethyl-1-(tert-butyl)-1H-indol-5-yl)guanadine

This compound was prepared according to the method of example 2b. A freshly prepared suspension of 3.95g 5-amino-2-ethyl-1-(tert-butyl)indole in ca. 200ml dimethylformamide and a suspension of 4.88g sodium dicyanamide were mixed and then 2.0ml 12N hydrochloric acid was added. The mixture was stirred at room temperature 12 hours and then heated at 50°C for 2 hours. The solvent was removed in vacuo to give a residue which was dissolved in methanol. Silica gel was added to the methanol solution and the solvent was removed in vacuo to give a silica gel suspension of the reaction mixture. The silica gel suspension was placed on a silica gel column and eluted with 50% ethyl acetate in hexane which yielded 2.2g of the expected product. NMR (DMSO-$d_6$): (δ 8.25 (s, 1H), 7.6 (d, 1H), 7.35 (s, 1H), 6.9 (d, 1H), 6.75 (br s, 2H), 6.2 (s, 1H), 3.0 (q, 2H), 1.8 (s, 9H), 1.3 (t. 3H).

g) 1,3-Diamino-7-(tert-butyl)-8-ethyl-7H-pyrrolo (3.2-f)quinazoline

This compound was prepared according to the method of Example 2c, method B. A suspension of 2.2g N-cyano-N'-(2-ethyl-1-(tert-butyl)-1H-indol-5-yl)guandidine in ca 300ml diglyme, bis (2-methoxyethyl) ether, was refluxed 40 hours. The solvent was removed in vacuo to give a residue which was taken up in methanol, silica gel was added and the solvent was removed in vacuo. The silica gel suspension of residue was placed on a silica gel column and eluted with 10% ethanol in chloroform to afford 1.30g product, m.p. 200-202°C, NMR (DMSO-$d_6$): δ 8.0 (d, 1H), 6.9 (d, 1H), 6.8 (s, 1H), 6.6 (br s, 2H), 5.6 (br s, 2H), 3.0 (q, 2H), 1.8 (s, 9H), 1.4 (t, 3H). λmax (0.1 N HC1): 347 (ε 10599); 231 (ε 29347)nm; λmin 289 (ε 1267) nm. Anal. Calcd for $C_{16}H_{21}N_5$.0.6 $H_2O$: C, 65.32; H, 7.61; N, 23.81. Found: C, 65.23;; H, 7.43; N, 23.69.

Example 6

1,3-Diamino-8-ethyl-7-(1-ethylpropyl)-7H-pyrrolo(3,2-f)quinazoline (Compound 6)

a) N-(1-ethylpropyl)-4-nitroaniline

A solution of 4-nitroaniline (13.8g) and concentrated hydrochloric acid (10ml) in methanol (200ml) was treated with a solution of 3-pentanone (14.5ml) in methanol (15ml) and stirred 1 hour at room temperature. The reaction was then cooled in an ice bath and a solution of sodium cyanoborohydride (8.7g) in methanol (35ml) was added so that the reaction temperature remained at less than 20°C. The reaction was then stirred at room temperature for 2 hours. The solution was made basic with 10° sodium hydroxide (70ml), partially concentrated in vacuo to remove most of the methanol and diluted to 350ml total volume with water. The aqueous solution was extracted with ether (2 x 250ml) and the organic extracts were dried with magnesium sulfate and concentrated in vacuo to give an oil. The oil was placed on a silica gel column and eluted with 40% hexane in methylene chloride to afford 17.3g of the product as an orange oil, NMR ($CDCl_3$): 8.06 (d, 2H), 6.50 (d, 2H), 4.20-4.30 (br s, 1H), 3.30-3.40 (m. 1H), 1.40-1.70 (m, 4H), 0.94 (t, 6H). Anal. Calcd for $C_{11}H_{16}N_2O_2$: C, 63.44; H, 7.74; N, 13.45. Found: C, 63.36; H, 7.72; N, 13.37.

b) N-(1-ethylpropyl)-2-iodo-4-nitroaniline

A mixture of 10.4g N-(1-ethylpropyl)-4-nitroaniline and 20ml concentrated hydrochloric acid in 150 ml water was heated to 50°C and treated dropwise over 1 hour with a solution of iodine monochloride (17g) in concentrated hydrochloric acid (30ml). The mixture was stirred at ca 50°C for 2.5 hours, cooled on an ice bath and treated with sodium sulfate (10g). The mixture was stirred 10 minutes at room temperature, and diethyl ether (125ml) was added and stirred an auditional 20 minutes. The aqueous layer was extracted with diethyl ether (100ml) and the combined organic extracts were washed with 100ml of saturated sodium bicarbonate solution,

dried with magnesium sulfate and concentrated in vacuo to give an oil. The oil was placed on an alumina column and eluted with 33% hexane in methylene chloride to afford 15.59g of an orange oil which was fractionally distilled to give 11.86g yellow oil, b.p. 170-172°C, NMR ($CDC1_3$): 8.57 (d, 1H), 8.10 (dd, 1H), 6.47 (d, 1H), 4.80 (br d, 1H) 3.40 (m, 1H), 1.50-1.75 (m, 4H), 0.96 (t, 6H). Anal. Calcd for $C_{11}H_{15}N_2O_2I$: C, 39.54; H, 4.52; N, 8.38. Found: C, 39.44; H, 4.50; N, 8.31.

c) 2-(1-butynyl)-N-(1-ethylpropyl)-4-nitroaniline

This compound was prepared according to the method of Example 5c. 8.0g N-(1-ethylpropyl)-2-iodo-4-nitroaniline afforded 6.8g of the product as an amber oil, NMR (DMSO-$d_6$): 8.0 (m, 2H), 6.8 (d, 1H), 5.7 (d, 1H), 3.5 (m, 1H), 2.5 (q, 2H), 1.6 (m, 4H), 1.2 (t, 3H), 0.8 (t, 6H). Anal. Calcd for $C_{15}H_{20}N_2O_2$: C, 69.20; H, 7.74; N, 10.76. Found: C, 69.30; H, 7.75; N, 10.73.

d) 2-Ethyl-1-(1-ethylpropyl)-5-nitro-1H-indole

This compound was prepared according to the method of Example 5d. Accordingly, 6.5g 2-(1-butyl)-N-(1-ethylpropyl)-4-nitroaniline afforded 6.0g yellow oil after chromatography. NMR (DMSO-$d_6$): 8.4 (s, 1H), 7.9 (d, 1H), 7.7 (d, 1H), 6.5 (s, 1H), 4.1-4.3 (m, 1H), 2.7-2.9 (m, 2H), 1.8-2.2 (m, 4H), 1.3 (t, 3H), 0.6 (t, 6H) Anal. Calcd for $C_{15}H_{20}N_2O_2$.0.1 $H_2O$: C, 68.73; H, 7.77; N, 10.69. Found: C, 68.55; H, 7.68; N, 10.80.

e) 5-Amino-2-ethyl-1-(ethylpropyl)indole hydrochloride

This compound was prepared according to the method of Example 2a and used immediately to prepare 6f.

f) N-cyano-N'-(2-ethyl-1-(1-ethylpropyl)-1H-indole-5-yl)guanadine

This compound was prepared according to the method of Example 2b using 6.1g 5-amino-2-ethyl-1-(1-ethylpropyl)indole hydrochloride. The crude product mixture was placed on a silica gel column and eluted with 50% hexane in ethyl acetate to provide a dark residue which was dissolved in 3ml methanol and added dropwise to rapidly stirring water (300ml) and stirred 1 hour. The mixture was then filtered to give 2.6g product. m.p. 145-147°C. NMR (DMSO-$d_6$): 8.8 (s, 1H), 7.5 (d, 1H), 7.4 (s, 1H), 6.9 (d, 1H), 6.8 (br s, 2H), 6.2 (br s, 1H), 4.1 (br m, 1H), 2.8 (q, 2H), 1.8-2.2 (m, 4H), 1.3 (t, 3H), 0.7 (t, 6H). Anal. Calcd for $C_{17}H_{23}N_5$: C, 68.66; H, 7.80; N, 23.55. Found: C, 68.41; H, 7.82; N, 23.35.

g) 1,3-Diamino-8-ethyl-7-(1-ethylpropyl)-7H-pyrrolo(3,2-f) quinazoline monohydrate

2.5g N-cyano-N'-(2-ethyl-1-(1-ethylpropyl)-1H-indole-5-yl) guanadine was dissolved in 50ml dimethoxyethane and heated to 60°C. Boron trifluoride etherate (3.2ml) was added and the mixture was stirred for 3 hours. The mixture was cooled and 300ml concentrated ammonium hydroxide in 500ml methanol was added. The mixture was stirred for 65 hours. The methanol was removed in vacuo and the mixture filtered to give 2.6g crude product. The crude product was placed on a silica gel column and eluted with 5% ethanol in ethyl acetate to give 2.5g light brown solid. The solid was stirred in 500ml 1N sodium hydroxide 18 hours and filtered to give 1.5g product, m.p. 155-157°C softens 115°C. NMR (DMSO-$d_6$): 7.8 (d, 1H), 7.0 (d, 1H), 6.8 (s, 1H), 6.7 (br s, 2H), 5.8 (br s, 2H), 4.2 (br m, 1H), 2.8 (q, 2H), 1.8-2.2 (m, 4H), 1.4 (t, 3H), 0.8 (t, 6H).
$\lambda$max (0.1 N HC1): 349 ($\varepsilon$ 11983), 231 ($\varepsilon$ 32393) nm; $\lambda$min 289 ($\varepsilon$ 1279), 218 ($\varepsilon$ 25652) nm. Anal. Calcd for $C_{17}H_{23}N_5 \cdot H_2O$: C, 64.74; H, 7.99; N, 22.20. Found: C, 64.77; H, 7.92; N, 22.13.

Example 7

The following compounds were prepared according to the procedure of example 1, starting from 1, 3-diamino-7H-pyrrolo(3,2-f)quinazoline:

a) 1,3-diamino-7-sec-butyl-7H-pyrrolo(3,2-f)quinazoline
b) 1,3-diamino-7-(1-methylbutyl)-7H-pyrrolo(3,2-f)quinazoline
c) 1,3-diamino-7-(1-ethyl-2-methylpropyl)-7H-pyrrolo(3,2-f)quinazoline
d) 1,3-diamino-7-isopropyl-7H-pyrrolo(3,2-f)quinazoline
e) 1,3-diamino-7-(2-methoxy-1-methoxymethyl)ethyl-7H-pyrrolo(3,2-f)quinazoline
f) 1,3-diamino-7-cyclohexyl-7H-pyrrolo(3,2-f)quinazoline

g) 1,3-diamino-7-(2-cyclohexen-1-yl)-7H-pyrrolo(3,2-f)quinazoline

Example 8

The followng compound was prepared according to the procedure of example 2, starting from 2-methyl indole:

1,3-diamino-7-isopropyl-8-methyl-7H-pyrrolo(3,2-f)quinazoline

Example 9

The following compounds were prepared according to the procedure of example 6, starting with 4-nitroaniline:

a) 1,3-diamino-7-(1-ethylpropyl)-8-(methoxymethyl)-7H-pyrrolo (3,2-f)quinazoline
b) 1,3-diamino-7-(1-ethylpropyl)-8-propyl-7H-pyrrolo(3,2-f)quinazoline
c) 1,3-diamino-8-tert-butyl-7-isopropyl-7H-pyrrolo(3,2-f)quinazoline
d) 1,3-diamino-7-(1-ethylpropyl)-8-isopropyl-7H-pyrrolo(3,2-f)quinazoline
e) 1,3-diamino-8-ethyl-7-sec-butyl-7H-pyrrolo(3,2-f)quinazoline
f) 1,3-diamino-8-(3-chloropropyl)-7-(1-ethylpropyl)-7H-pyrrolo(3, 2-f)quinazoline.

Example 10

The following compounds were prepared according to the procedure of example 5, using 4-fluoro-nitrobenzene:

a) 1,3-diamino-8-butyl-7-tert-butyl-7H-pyrrolo(3,2-f)quinazoline
b) 1,3-diamino-7-(1,1-dimethylpropyl)-8-methyl-7H-pyrrolo(3,2-f)quinazoline

a = NMR data
b = mass spectrum data
c = melting point (°C)

No.

1

a) δ 7.85 (d, 1H, aromatic); 7.55 (d, 1H, aromatic); 7.1 (d, 1H, aromatic); 7.0 (d, 1H, aromatic); 6.8 (br s, 2H, $NH_2$); 5.9 (br s, 2H, $NH_2$); 4.3 (m, 1H); 1.8 (m, 4H); 0.6 (t, 6H)
b) 270 ($M^+$ +1, 100%)
c) 197-198

2

a) δ 7.8 (d, 1H, aromatic); 6.9 (d, 1H, aromatic); 6.8 (s, 1H, aromatic); 6.6 (br s, 2H, $NH_2$); 5.6 (br s, 2H, $NH_2$); 4.1 (br m, 1H); 2.4 (br s, 3H); 2.1 (br m, 2H); 1.9 (m, 2H); 0.6 (t, 6H)
b) 284 ($M^+$ +1, 100%)
c) 200-201

3

a) δ 7.8 (d, 1H, aromatic); 7.5 (d, 1H, aromatic); 7.1 (d, 1H, aromatic); 7.0 (d, 1H, aromatic); 6.65 (br s, 2H, $NH_2$); 5.7 (br s, 2H, $NH_2$); 4.4 (m, 1H); 1.8 (m, 4H); 1.0 (m, 2H); 0.8 (t, 3H); 0.6 (t, 3H)
b) 284 ($M^+$ +1, 100%)
c) 197-198

4

a) δ 12.7 (br s, 1H); 8.8 (br s, 1H); 8.2 (d, 1H, aromatic); 7.8 (d, 1H, aromatic); 7.5 (br s, 2H, $NH_2$); 7.4 (d, 1H, aromatic), 7.2 (d, 1H, aromatic); 4.6 (m, 1H); 1.8 (m, 4H); 1.1 (m, 2H, partially overlapping with other aliphatic); 0.84 (m, 2H, partially overlapping with other aliphatic); 0.73-0.79 (m, 6H)
b) 298 ($M^+$ +1, 100%)
c) >250

5

a) δ 8.0 (d, 1H, aromatic); 6.95 (d, 1H, aromatic); 6.8 (s, 1H, aromatic); 6.6 (br s, 2H, $NH_2$); 5.6 (br s, 2H, $NH_2$); 3.1 (q, 2H); 1.8 (s, 9H); 1.4 (t, 3H).
b) 284 ($M^+$ +1, 100%)
c) 200-202

6

a) δ 7.8 (d, 1H, aromatic); 6.95 (d, 1H, aromatic); 6.8 (s,1H, aromatic, partially buried by $NH_2$ peak); 6.75 (br s, 2H, $NH_2$); 5.8 (br s, 2H, $NH_2$); 4.2 (m, 1H); 2.8 (q, 2H); 1.8-2.2 (m, 4H); 1.4 (t, 3H); 0.6 (t,

6H).
b) 298 ($M^+$ +1, 100%)
c) 155-157

7a

a) δ 7.8 (d, 1H, aromatic); 7.5 (d, 1H, aromatic); 7.0 (d, 1H, aromatic); 7.0 (d, 1H, aromatic, partially buried by the other aromatic); 6.7 (br s, 2H, $NH_2$); 5.7 (br s, 2H, $NH_2$); 4.5 (m, 1H); 1.8 (m, 2H); 1.5 (d, 3H); 0.7 (t, 3H)
b) 256 ($M^+$ +1, 100%)
c) 188-189

7b

a) δ 7.8 (d, 1H, aromatic); 7.55 (d, 1H, aromatic); 7.05 (d, 1H, aromatic); 6.6 (br s, 2H, $NH_2$); 5.6 (br s, 2H, $NH_2$); 4.7 (m, 1H); 1.7-1.9 (m, 2H); 1.5 (d, 3H); 0.9-1.3 (m, 2H); 0.08 (t,3H)
b) 270 ($M^+$ +1, 100%)
c) 118-119

7c

a) δ 7.8 (d, 1H, aromatic); 7.5 (d, 1H, aromatic); 7.1 (d, 1H, aromatic); 7.0 (d, 1H, aromatic); 6.6 (br s, 2H, $NH_2$); 5.6 (br s, 2H, $NH_2$); 4.1 (m, 1H); 1.7-2.2 (m, 3H); 1.0 (d, 3H); 0.52-0.64 (m, 6H)
b) 284 ($M^+$ +1, 100%)
c) 118-120

7d

a) δ 7.9 (d, 1H, aromatic); 7.7 (d, 1H, aromatic); 7.0-7.2 (d, 1H, aromatic, partially buried by the $NH_2$ peak, d, 1H, aromatic, buried by the $NH_2$ peak, br s, 2H, $NH_2$); 6.2 (br s, 2H, $NH_2$); 4.8 (multiplet, 1H); 1.5 (d, 6H)
b) 242 ($M^+$ +1, 100%)
c) 228-231

7e

a) δ 7.8 (d, 1H, aromatic); 7.6 (d, 1H, aromatic); 7.4 (d, 1H, aromatic); 7.1 (d, 1H, aromatic); 7.0 (d, 1H, aromatic, partially buried by the other aromatic); 6.7 (br s, 2H, $NH_2$); 5.7 (br s, 2H, $NH_2$); 4.9 (m, 1H); 3.7 (d, 2H); 3.7 (d, 2H, partially buried); 3.2 (s, 6H)
b) 302 ($M^+$ +1, 100%)
c) 148-150

7f

a) δ 7.9 (d, 1H, aromatic); 7.6 (d, 1H, aromatic); 7.0-7.1 (m, 2H, aromatic); 6.9 (br s, 2H, $NH_2$); 6.0 (br s, 2H, $NH_2$); 4.4 (m, 1H); 1.1-2.0 (m, 10H)
b) 282 ($M^+$ +1, 100%)
c) 147-150

7g

a) δ 7.8 (d, 1H, aromatic); 7.3 (d, 1H, aromatic); 7.0-7.1 (m, 2H, aromatic); 6.6 (br s, 2H, $NH_2$); 5.7-6.2 (m, 2H, CH=CH); 5.6 (br s, 2H, $NH_2$); 5.2 (br s, 1H); 1.6-2.3 (m, 6H)
b) 280 ($M^+$ +1, 100%)
c) 237-239

8a

a) δ 7.8 (d, 1H, aromatic); 6.9 (d, 1H, aromatic); 6.8 (s, 1H, aromatic); 6.7 (br s, 2H, $NH_2$); 5.7 (br s, 2H, $NH_2$); 4.8 (m, 1H); 2.5 (s, 3H); 1.5 (d, 6H)
b) 256 ($M^+$ +1, 100%)
c) 150-160 (dec)

9a

a) δ 7.9 (d, 1H, aromatic); 7.1 (s, 1H, aromatic, partially buried by the other aromatic); 7.0 (d, 1H, aromatic); 6.8 (br s, 2H, $NH_2$); 5.9 (br s, 2H, $NH_2$); 4.6 (s, 2H); 4.2 (br m, 1H); 3.3 (s, 3H, partially buried by the solvent water peak); 2.1 (br m, 2H); 1.9 (br m, 2H); 0.6 (m, 6H)
b) 314 ($M^+$ +1, 100%)
c) 159-161

9b

a) δ 7.78 (d, 1H, aromatic); 6.91 (d, 1H, aromatic); 6.79 (s, H, aromatic); 6.60 (br s, 2H, $NH_2$); 5.6 (br s, 2H, $NH_2$); 4.15 (br m, 1H); 2.74 (t, 2H); 2.10 (m, 2H); 1.9 (m, 2H); 1.75 (m, 2H); 1.03 (t, 3H); 0.65 (t, 6H)
b) 312 ($M^+$ +1, 100%)
c) 121-122

9c

a) δ 7.87 (d, 1H, aromatic); 6.96 (d, 1H, aromatic); 6.67 (s, 1H); 6.60 (br s, 2H, $NH_2$); 5.6 (br s, 2H, $NH_2$); 5.15 (m, 1H); 1.62 (d, 6H); 1.47 (s, 9H)

b) 298 ($M^+$ +1, 100%)

c) 286-287

9d

a) δ 7.8 (d, 1H, aromatic); 6.95 (d, 1H, aromatic); 6.85 (s, 1H, aromatic); 6.6 (br s, 2H, $NH_2$); 5.6 (br s, 2H, $NH_2$); 4.2 (m, 1H); 3.2 (m, 1H, partially buried by the solvent water peak); 2.0 (m, 4H); 1.3 (d, 6H); 0.7 (t, 6H).

b) 312 ($M^+$ +1, 100%)

c) 243-244

9e

a) δ 7.8 (d, 1H, aromatic); 7.0 (d, 1H, aromatic); 6.8 (s, 1H, aromatic); 6.6 (br s, 2H, $NH_2$); 5.6 (br s, 2H, $NH_2$); 4.4 (br m, 1H); 2.8 (q, 2H); 1.8-2.2 (m, 2H); 1.6 (d, 3H); 1.35 (t, 3H); 0.7 (t, 3H)

b) 284 ($M^+$ +1, 100%)

c) 199-200

9f

a) δ 7.8 (d, 1H, aromatic); 6.95 (d, 1H, aromatic); 6.85 (s, 1H, aromatic); 6.8 (br s, 2H, $NH_2$); 5.75 (br s, 2H, $NH_2$); 4.2 (m, 1H); 3.8 (t, 2H); 2.9 (t, 2H); 1.8-2.4 (m, 6H); 0.6 (t, 6H).

b) 346 ($M^+$ +1, 100%)

c) 174-175

10a

a) Trifluoracetic acid salt: 1H: d 8.3 (d, 1H, aromatic); 7.3 (br s, 2H, $NH_2$); 7.1 (d, 1H, aromatic); 7.0 (s, 1H, aromatic); 3.0 (m, 2H); 1.8 (s, 9H); 1.7 (m, 2H); 1.4 (m, 2H); 0.9 (t, 3H) p.p.m. $19_F$: δ 34000.

b) 312 ($M^+$ +1, 100%)

c) 233-234

10b

a) δ 8.0 (d, 1H, aromatic); 6.9 (d, 1H, aromatic); 6.8 (s, 1H, aromatic); 6.6 (br s, 2H, $NH_2$); 5.6 (br s, 2H, $NH_2$); 2.65 (s, 3H); 2.15 (q, 2H); 1.8 (s, 6H); 0.6 (t, 3H).

b) 284 ($M^+$ +1), 100%)

c) 254-256

The following examples illustrate pharmaceutical formulations according to the present invention:-

Injectable solution

A solution for intramuscular injection may be prepared by mixing:-

| | |
|---|---|
| Compound of formula (II) | 9.5 parts by weight |
| Dimethyl sulphoxide | 19.0 parts by weight |
| Sorbitan monooleate | 4.5 parts by weight |
| Corn oil | 67.0 parts by weight |
| | 100.0 |

Injectable solution

| | |
|---|---|
| Compound of formula (II) | 5 parts by weight |
| N-methyl-pyrrolidone | 48.3 parts by weight |
| Tween 80 | 2 parts by weight |
| Span 80 | 4.7 parts by weight |
| Miglyol 812 | 40 parts by weight |
| | 100.0 |

Tablet

| | |
|---|---|
| Compound of formula (II) | 25.0 mg |
| Lactose BP | 48.5 mg |
| Microcrystalline Cellulose BP ("Avicel pH 101") | 10.0 mg |
| Low-substituted Hydroxypropyl; Cellulose BP ("LHPC LH-11") | 10.0 mg |
| Sodium Starch Glycollate BP ("Explotab") | 3.0 mg |
| Povidone BP ("K30") | 3.0 mg |
| Magnesium Stearate BP | 0.5 mg |
| | 100.0 mg |

Oral suspension

| | |
|---|---|
| Compound of formula (II) | 50 mg |
| Avicel RC 591 | 75 mg |
| Sucrose syrup | 3.5 ml |
| Methylhydroxybenzoate | 5 mg |
| Colour | 0.01% w/v |
| Cherry flavour | 0.1% v/v |
| Tween 80 | 0.2% v/v |
| Water | to 5 ml |

Injectable suspension

| | |
|---|---|
| Compound of formula (II) | 100 mg |
| Polyvinyl pyrrolidone (PVP) | 170 mg |
| Tween 80 | 0.2% v/v |
| Methylhydroxybenzoate | 0.1% v/v |
| Water for Injection | to 3 ml |

Capsule

| | |
|---|---|
| Compound of formula (II) | 100 mg |
| Starch 1500 | 150 mg |
| Magnesium stearate filled into a hard gelatin capsule | 2.5 mg |

Suspension for Nebulisation

| | |
|---|---|
| Compound of formula (II), sterile | 1.0 mg |
| Water for Injections | to 10.0 ml |

Disperse the compound of formula (II) in the Water for Injections previously sterilised in a sterile container. Fill into sterile glass ampoules, 10ml/ampoule under aspetic conditions, and seal each ampoule by fusion of the glass.

Aerosol Formulation

| | |
|---|---|
| Compound of formula (II), micronised | 1.0 mg |
| Aerosol propellant to | 5.0 mg |

Suspend the micronised compound of formula (II) in the aerosol propellant. Fill this suspension into preformed aerosol cannisters, 5ml/cannister under pressure, through the valve orifice.

Powder Inhalation

| | |
|---|---|
| Compound of formula (II), micronised | 1.0 mg |
| Lactose | 29.0 mg |

Triturate and blend the micronised compound of formula (II) with the lactose. Fill the resulting powder blend into hard gelatin capsule shells, 30mg per capsule.

Nasal Drops

| | |
|---|---|
| Compound of formula (II) | 100.0 mg |
| Methylhydroxybenzoate | 10.0 mg |
| Water for Injections | to 10.0 mg |

Disperse the compound of formula (II) and the methylhydroxybenzoate in the Water for Injections. Fill this suspension into suitable dropper bottles, 10ml/bottle, and close by securing the dropper nozzle and bottle cap.

Transdermal Solution

0.01-0.2M solution of the active compound in a citrate or bis/tris buffer (pH 6) or ethanol/water.

Ointments

| (A) | Ingredients | 1% Anhydrous Ointment Amount per 100 g |
|---|---|---|
| | Compound of formula (II)<br>Petrolatum, White | 1.0 g<br>q.s. |
| | | 100.0 g |

White petrolatum is melted by heating to 45°C-60°C. Compound of formula (II) is added and dispersed with aid of a mixer. The ointment is then cooled to room temperature.

| (B) | Ingredients | 10% Anhydrous Ointment Amount per 100 g |
|---|---|---|
| | Compound of formula (II)<br>Petrolatum, White | 10.0 g<br>q.s. |
| | | 100.0 g |

(Preparation as described in (A) above).

In vitro activity vs human dihydrofolate reductase enzyme

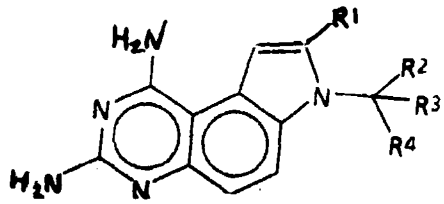

| Compound | $R^1$ | $R^2$ | $R^3$ | $R^4$ | DHFR $I_{50}$ (X $10^{-8}$M) | |
|---|---|---|---|---|---|---|
| | | | | | C.albicans | Human |
| 1 | H | Et | Et | H | 0.45 | <0.1 |
| 2 | Me | Et | Et | H | <0.1 | <0.1 |
| 3 | H | n-Pr | n-Et | H | 0.2 | <0.1 |
| 4 | H | n-Pr | n-Pr | H | <0.1 | <0.2 |
| 5 | Et | Me | Me | Me | <0.1 | <0.1 |
| 6 | Et | Et | Et | H | <0.1 | <0.1 |
| 7a | H | Me | Et | H | 1.0 | 1.8 |
| 7b | H | Me | n-Pr | H | 0.70 | 1.1 |
| 7c | H | Et | i-Pr | H | 0.30 | <0.2 |
| 7d | H | Me | Me | H | 3.5 | 4.8 |
| 7e | H | $CH_2OCH_3$ | $CH_2OCH_3$ | H | 0.89 | 0.84 |
| 7f | H | $-CH_2CH_2CH_2CH_2CH_2-$ | | H | 0.30 | 4.4 |
| 7g | H | $-CH_2CH_2CH_2CH=CH-$ | | H | 0.60 | 3.3 |
| 8a | Me | Me | Me | H | 0.40 | 0.90 |
| 9a | $CH_2OCH_3$ | Et | Et | H | 0.41 | 0.33 |
| 9b | n-Pr | Et | Et | H | 0.18 | 0.13 |
| 9c | t-Bu | Me | Me | H | <0.1 | <0.1 |
| 9d | i-Pr | Et | Et | H | <0.1 | <0.1 |
| 9e | Et | Me | Et | H | <0.1 | <0.1 |
| 9f | $CH_2CH_2CH2$ Cl | Et | Et | H | 0.43 | <0.1 |
| 10a | n-Bu | Me | Me | Me | 0.70 | 3.9 |
| 10b | Me | Et | Me | Me | <0.1 | <0.1 |

Method

The enzyme binding affinities of human dihydrofolate reductase [Prendergast et al, (1988) Biochemistry, 27, 3634-3671] for Compound 1 and Compound 2 were determined with minor modifications of the method previously described [Baccanari and Joyner, (1981) Biochemistry, 20, 1710-1716]. Briefly, the $K_I$ values were determined in 50mM Sorenson's phosphate buffer, pH 7 using 45μM dihydrofolate and 65μM NADPH, and the

value of 1250 used for the expression (1 + $S/K_s$) in the Henderson equation was based on a dihydrofolate $K_s$ value of 0.036μM [Delcamp et al, (1983) Biochemistry, 22, 633-639].

Results

| Compound | human dihydrofolate reductase $K_i$ (pM) |
|---|---|
| 1 | 19 |
| 2 | 1.4 |
| Methotrexate | 1.8 |

In vitro activity vs mouse or human cell lines

Method

The cytotoxic effects of various compounds were measured by the inhibition of mammalian cell growth in culture. Cell lines were grown in RPMI-1640 media prepared without folic acid but supplemented with 10nM calcium leucovorin, 25mM HEPES and 10% dialysed fetal bovine serum. In the case of human medulloblastoma cells (Daoy), the media (containing undialysed 10% fetal bovine serum) was pretreated with the enzyme thymidine phosphorylase (20 units/ml for 30 mins. at 37°C) to remove endogenous thymidine.

Cells were seeded into 96 well microtiter plates (at densities determined to give log phase growth of control cells at the end of the assay) in 150μl of medium and grown at 37°C in a humidified incubator at 6% $CO_2$ for 24 hours. Compounds were prepared in media at four times the highest concentration to be tested; two-fold serial dilutions were made, and compounds were added to cells. After a further 72 hours at 37°C in a humidified incubator at 6% $CO_2$, cell growth was measured using either the sulforhodamine 8 protein stain assay [Skehan et al (1990), JNCI, 82, 1007-1112] for monolayer cultures or the MTT dye reduction assay [Carmichael et al (1987), Cancer Res. 47, 936-942] for suspension cultures. The $EC_{50}$, defined as the concentration of compound required to reduce growth to 50% of the control, was determined from a plot of per cent inhibition versus log concentration.

Results

Table 1. COMPARATIVE CYTOTOXICITIES IN CELL CULTURE

| Cell line | $EC_{50}$(nM) | | | |
|---|---|---|---|---|
| | Compound 1 | Compound 2 | Piritre-xim | Methotre-xate |
| MOUSE LINES | | | | |
| S180 sarcoma | 4.8 | | 10 | 18.7 |
| Ehrlich ascites carcinoma | 1.9 | | 4 | 8.5 |
| P388 lymphoid neoplasm | 2.7 | 0.3 | 23 | 2.7 |
| L cells (derived from connective tissue) | 2.2 | 0.7 | 5.2 | 75 |
| HUMAN LINES | | | | |
| KB3-1 epidermoid carcinoma | 70 | 13 | 340 | 20 |
| HCT8 colon adenocarcinoma | 1.2 | 0.3 | 1.3 | 7.3 |
| GC3C1 colon carcinoma | 3.5 | 0.8 | 7.6 | 12 |
| MCF7 breast adenocarcinoma | 38 | 0.9 | 175 | 12 |
| D98 bone marrow cells | 45 | 12 | 195 | 47 |
| CCRF-CEM T cells | 0.7 | <1 | 15 | 1.9 |
| Daoy medulloblastoma | 8.4 | 0.4 | 48 | 21 |

Cells were continuously exposed to inhibitor for 72 hours.

In vitro activity vs microorganisms

Method

The minimum inhibtory concentration (MIC) is defined as the lowest concentration of drug that inhibits 90% of bacterial growth during 18 hours of incubation at 37°C. The culture medium used in the test was Wellcotest broth to which lysed horse blood had been added at a final concentration of 7%. The assay was semi-automated employing 96-well Microtiter plates, and growth was monitored visually.

Results

Medium: WELLCOTEST SENSITIVITY TEST BROTH PLUS 7% LYSED HORSE BLOOD

| ORGANISM | | M I C - μG/ML | | | |
|---|---|---|---|---|---|
| | | Compound 1 | Trimethoprim | Compound 2 | Trimethoprim |
| Str. Pyogenes | CN10 | 0.1 | 0.1 | .0001 | 0.1 |
| Str. Faecalis | CN478 | 0.1 | 0.1 | .0001 | 0.1 |
| Str. Agalactiae | CN1143 | 0.1 | 0.1 | .0001 | 0.1 |
| Staph. Aureus | CN491 | 0.1 | 0.1 | 0.01 | 0.1 |
| Bord. Bronchiseptica | CN385 | 0.1 | 0.3 | 0.003 | 1.0 |
| Vibrio Cholerae | ATCC14035 | - | - | .0001 | 0.3 |
| Past. Multocida | ATCC6587 | 0.1 | 0.1 | .0001 | 0.1 |
| Candida Albicans | CN1863 | 0.3 | >100 | 0.03 | >100 |
| Myco. Smegmatis | S3254 | 0.1 | 0.3 | 0.001 | 0.3 |
| Sal. Typhosa | CN512 | 0.1 | 0.1 | 0.1 | 0.1 |
| Sal. Typhimurium | S8587 | 0.1 | 0.1 | 0.003 | 0.1 |
| Shig. Flexneri | CN6007 | 0.1 | 0.1 | 0.003 | 0.1 |

| | | | | | |
|---|---|---|---|---|---|
| Esch. Coli | CN314 | 0.1 | 0.1 | .0001 | 0.1 |
| Serr. Marcescens | CN8712 | 0.1 | 0.3 | 0.03 | 1.0 |
| Kleb. Pneumoniae | CN3632 | 0.3 | 0.1 | 0.03 | 0.3 |
| Entero. Aerogenes | S3770 | 0.1 | 0.1 | 0.01 | 0.1 |
| Citro. Freundii | P1436 | 0.1 | 0.1 | 0.3 | 0.1 |
| Pr. Vulgaris | CN329 | 0.1 | 0.1 | 0.01 | 1.0 |
| Pr. Mirabilis | S2409 | 0.3 | 3.0 | 0.1 | 1.0 |
| Ps. Aeruginosa | CN200 | 1.0 | 100 | 3.0 | 100 |

NOTE: These compounds were also tested for in vitro activity against the following microorganisms grown in glucose minimal medium (GMM): Sal. Typhimurium LT2 (S8587), Esch. Coli ML30, Ps. Aeruginosa (CN200), Ps. Aeruginosa (S5641), and B. Megaterium (CN2623). There was essential agreement between these MICs and the ones derived from organisms grown in Wellcotest Broth plus 7% Lysed Horse Blood.

Brain Distribution of Compounds 1 and 2

Method

Female CD1 mice were dosed with Compound 1 (17mg/kg, i.v.), Compound 2 (10mg/kg, i.v.), piritrexim (200mg/kg,i.p.) or trimetrexate (100mg/kg, i.p.). Brain and plasma samples were collected 30 or 40 minutes later. The drugs were extracted from the samples, and their concentrations were determined using HPLC. The numbers represent the average of several experiments.

Results

| Compound | Tissue concentration | | Ratio |
|---|---|---|---|
| | brain (μg/g) | plasma (μg/ml) | brain/plasma |
| 1 | 5.0 | 0.70 | 7.0 |
| 2 | 2.4 | 0.36 | 6.7 |
| Piritrexim | 2.3 | 4.2 | 0.55 |
| Trimetrexate | 1.4 | 13 | 0.10 |

In vivo activity against brain implants of tumour cells in mice

Method

Intracranial implants of 1 x $10^5$ P388 murine leukemia cells were made in female CD2F1 mice, then the animals were monitored for survival with or without treatment with Compound 1 (40mg/kg, s.c., days 1-10) or Compound 2 (10mg/kg, s.c., days 1-10). Both compounds were suspended in 0.5% carboxymethylcellulose and 1% Tween 80 and injected in a volume of 0.2ml/20g. Therapeutic response was monitored by comparing the median day of death of the treated animals to that of the untreated controls.

Results

Mice treated with either Compound 1 or Compound 2 experienced a 50% to 55% increase in lifespan compared to controls.

A. In vitro activity vs Mycobacterium avium complex (MAC):

Method:

Compounds are tested for anti-MAC activity using a radiometric respirometry method (BACTEC, Johnston Laboratories, Towson, MD). Briefly, the principle of the assay is as follows: The growth medium 7H12 broth (Johnston Laboratories), contains $^{14}C$- labeled substrate (palmitic acid) as a single source of carbon. Growth leads to the metabolism of the substrate with subsequent release of $^{14}CO_2$ into the sealed vial, which is then measured by the BACTEC 301B instrument. The result is expressed as a "growth index" (GI) on a scale from 0 to 1000. Two drug-free controls are included for each organism: one vial is inoculated the same as the drug-containing test viels, and the second is inoculated with a 1:10 dilution of the original inoculum to represent 10% of the bacterial population. The $MIC_{90}$ of a compound is considered to be the level of drug which allows a daily GI increase equal to that of the 1:10 control.

The range of growth inhibition for compound 2 against seven (7) clinical isolates of Myco. avium is 97-99% at 5μg/ml and 69-97% at 1μg/ml. This roughly translates to an average MIC of 1.6 μg/ml.

B. In vitro activity vs Mycobacterium tuberculosis: (TB)

Method

Compounds which have been sent to the Centers for Disease Control for anti-TB and anti-MAC activity are tested using the "proportion method". This method is described in the CDC publication, "Public Health Mycobacteriology," P.Kent and G.Kubica, 1985. Briefly, the proportion method consists of plating equal quantities of several dilutions of inoculum on both control and drug-containing media (Middlebrook 7H10 agar supplemented with OADC enrichment). Test plates are incubated in a 5-10% $CO_2$ atmosphere at 37°C and read weekly for 3 weeks.

Resistance or susceptibility is determined by comparing the number of colonies that grow on the drug-containing medium with the number grown on the control medium. The MIC is considered to be the lowest concentration of drug which inhibits at least 90% of the organisms.

The average MIC for compound 2 against ten(10) clinical isolates of Myco. tuberculosis (including several multiply-resistant strains) is 3.2 μg/ml.

SCHEME 1

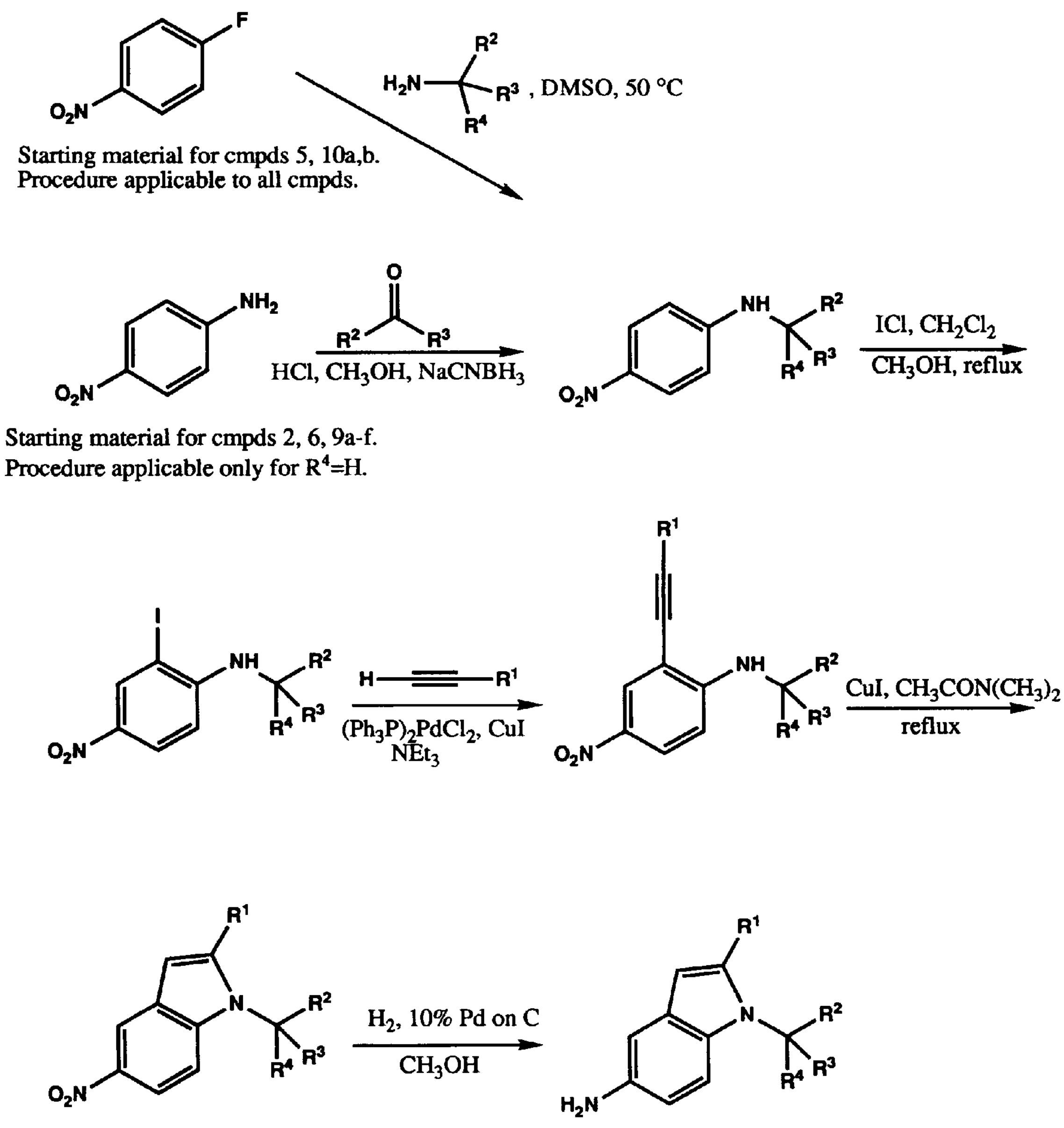
, DMSO, 50 °C
Starting material for cmpds 5, 10a,b.
Procedure applicable to all cmpds.
HCl, CH3OH, NaCNBH3
Starting material for cmpds 2, 6, 9a-f.
Procedure applicable only for R4=H.
ICl, CH2Cl2
CH3OH, reflux
(Ph3P)2PdCl2, CuI
NEt3
CuI, CH3CON(CH3)2
reflux
H2, 10% Pd on C
CH3OH

SCHEME 2

Procedure applicable only for $R^4$=H.
Not applicable to cmpds 7e, 9a,c,f.

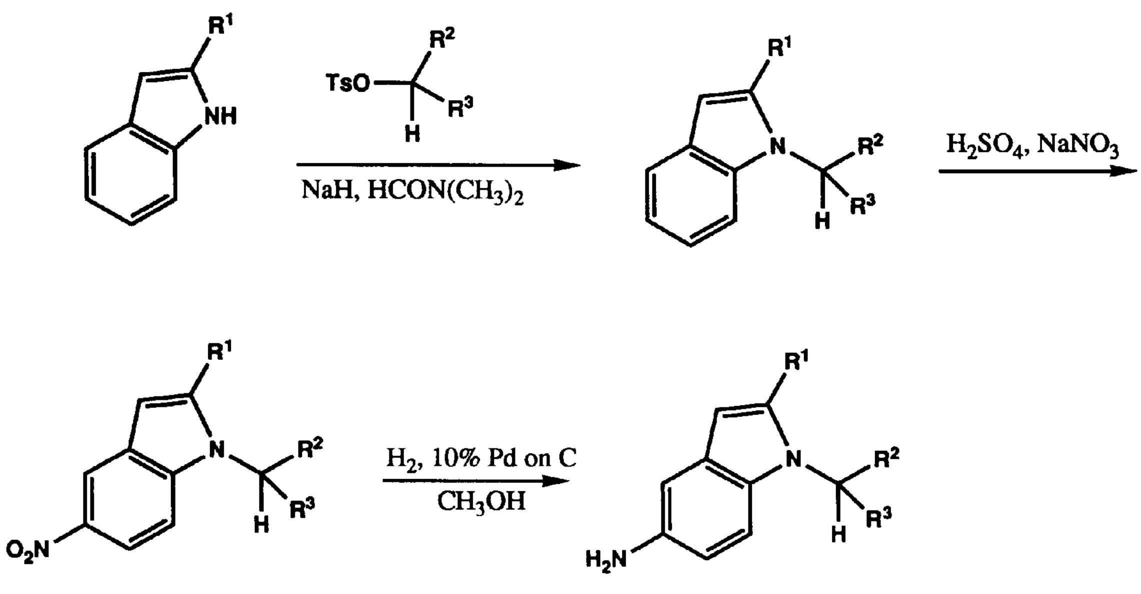


## Claims

1) A compound of the formula (II):

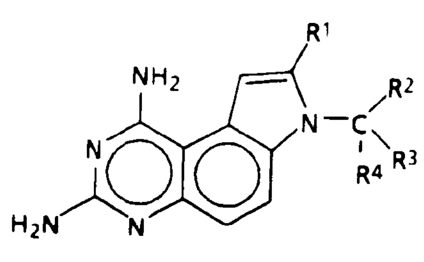

(II)

or an acid addition salt thereof, wherein $R^1$ is hydrogen, $C_{1-6}$ alkyl or $C_{1-4}$ alkyl substituted by halo or $C_{1-4}$ alkoxy, $R^2$ is $C_{1-4}$ alkyl or $C_{1-4}$ alkyl substituted by halo or $C_{1-4}$ alkoxy, $R^3$ is $C_{1-4}$ alkyl or $C_{1-4}$ alkyl substituted by halo

or $C_{1-4}$ alkoxy or

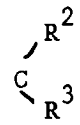

forms a $C_{5-7}$ cycloalkyl or cycloalkenyl group and $R^4$ is hydrogen or $C_{1-4}$ alkyl.

**2)** A compound of the formula (II) according to claim 1, wherein $R^1$ is hydrogen, methyl, ethyl, i-propyl, sec-butyl, t-butyl; $R^2$ is ethyl or n-propyl; $R^3$ is ethyl; and $R^4$ is hydrogen or methyl.

**3)** A compound of the formula (IIa)

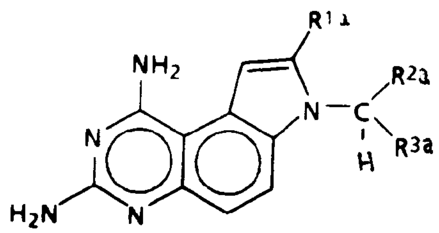

(IIa)

or an acid addition salt thereof, wherein $R^{1a}$ is hydrogen or $C_{1-4}$ alkyl, $R^{2a}$ is $C_{1-4}$ alkyl, and $R^{3a}$ is $C_{1-4}$ alkyl.

**4)** A compound selected from:

1.3-diamino-7-(1-ethylpropyl)-7H-pyrrolo(3,2-f)quinazoline
1,3-diamino-7-(1-ethylpropyl)-8-methyl-7H-pyrrolo(3,2-f)quinazoline
1,3-diamino-7-(1-ethylbutyl)-7H-pyrrolo(3,2-f)quinazoline
1,3-diamino-7-(1-propylbutyl)-7H-pyrrolo(3,2-f)quinazoline
1,3-diamino-7-(tert-butyl)-8-ethyl-7H-pyrrolo(3,2-f)quinazoline
1,3-diamino-8-ethyl-7-(1-ethylpropyl)-7H-pyrrolo(3,2-f)quinazoline
1,3-diamino-7-sec-butyl-7H-pyrrolo(3,2-f)quinazoline
1,3-diamino-7-(1-methylbutyl)-7H-pyrrolo(3,2-f)quinazoline
1,3-diamino-7-(1-ethyl-2-methylpropyl)-7H-pyrrolo(3,2-f)quinazoline
1,3-diamino-7-isopropyl-7H-pyrrolo(3,2-f)quinazoline
1,3-diamino-7-(2-methoxy-1-(methoxymethyl)ethyl)-7H-pyrrolo(3,2- f)quinazoline
1,3-diamino-7-cyclohexyl-7H-pyrrolo(3,2-f)quinazoline
1,3-diamino-7-(2-cyclohexen-1-yl)-7H-pyrrolo(3,2-f)quinazoline
1,3-diamino-7-isopropyl-8-methyl-7H-pyrrolo(3,2-f)quinazoline
1,3-diamino-7-(1-ethylpropyl)-8-(methoxymethyl)-7H-pyrrolo(3,2-f)quinazoline
1,3-diamino-7-(1-ethylpropyl)-8-propyl-7H-pyrrolo(3,2-f)quinazoline
1,3-diamino-8-(tert-butyl)-7-isopropyl-7H-pyrrolo(3,2-f)quinazoline
1,3-diamino-7-(1-ethylpropyl)-8-isopropyl-7H-pyrrolo(3,2-f)quinazoline
1,3-diamino-8-ethyl-7-sec-butyl-7H-pyrrolo(3,2-f)quinazoline
1,3-diamino-8-(3-chloropropyl)-7-(1-ethylpropyl)-7H-pyrrolo(3, 2-f)quinazoline.
1,3-diamino-8-butyl-7-(tert-butyl)-7H-pyrrolo(3,2-f)quinazoline
1,3-diamino-7-(1,1-dimethylpropyl)-8-methyl-7H-pyrrolo(3,2-f)quinazoline

**5)** A process for the preparation of a compound of the formula (II) according to any one of claims 1-3 which comprises:

(i) when $R^4$ is hydrogen, the alkylation of 7H-pyrrolo[3,2-f] quinazoline-1,3-diamine or an 8-alkyl derivative thereof, with the appropriate reagent $ZCHR^2R^3$, wherein $R^2$ and $R^3$ are as defined in relation to any one of claims 1-3 and Z is a leaving group, in the presence of a strong base.

(ii) the cyclisation of a compound of the formula (III).

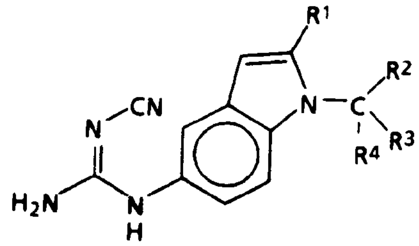

(III)

wherein $R^1$, $R^2$, $R^3$ and $R^4$ are as defined in relation to any one of claims 1-3.

**6)** A pharmaceutical formulation which comprises a compound of the formula (II) or a pharmaceutically acceptable salt thereof as defined in relation to any one of claims 1 to 4, and a pharmaceutically acceptable carrier therefor.

**7)** A compound of the formula (II) or a pharmaceutically acceptable salt thereof, according to any one of claims 1 to 4, for use in medicine.

**8)** The use of a compound of the formula (II), or a pharmaceutically acceptable salt thereof, as defined according to any one of claims 1 to 4 for the manufacture of a medicament for the treatment of neoplastic growth.

**9)** The use of a compound of the formula (II), or a pharmaceutically acceptable salt thereof, as defined according to any one of claims 1 to 4 for the treatment of disorders of the immune system.

**10)** The use of a compound of the formula (II), or a pharmaceutically acceptable salt thereof, as defined according to any one of claims 1 to 4 for the treatment of bacterial infections in mammals.

**11)** The use of a compound of the formula (II), or a pharmaceutically acceptable salt thereof, as defined according to any one of claims 1 to 4 for the treatment of protozoal infections in mammals.

**12)** The use of a compound of the formula (II), or a pharmaceutically acceptable salt thereof, as defined according to any one of claims 1 to 4 for the treatment of fungal infections in mammals.

**13)** A method for the treatment of susceptible malignant tumours in an animal which comprises administering to the animal a therapeutically effective amount of a compound of the formula (II) or a pharmaceutically acceptable salt thereof, as defined in relation to any one of claims 1 to 4.

**14)** A method for the treatment of disorders of the immune system in an animal which comprises administering to the animal a therapeutically effective amount of a compound of the formula (II) or a pharmaceutically acceptable salt thereof, as defined in relation to any one of claims 1 to 4.

**15)** A method for the treatment of bacterial protozoal or fungal infections in an animal which comprises administering to the animal a therapeutically effective amount of a compound of the formula (II) or a pharmaceutically acceptable salt thereof, as defined in relation to any one of claims 1 to 4.

European Patent Office

# PARTIAL EUROPEAN SEARCH REPORT

which under Rule 45 of the European Patent Convention shall be considered, for the purposes of subsequent proceedings, as the European search report

Application Number

EP 92 31 0232

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 208 520 (K.W. LEDIG ET AL.)<br>* the whole document *<br>--- | 1-12 | C 07 D 487/04<br>A 61 K 31/505 |
| A,D | US-A-4 118 561 (K.W. LEDIG)<br>* the whole document *<br>--- | 1-12 | |
| A | E.A. POTTERTON ET AL. 'Chem. Biol. Pteridines, Proc. Int. Symp. Pteridines Folic Acid Deriv. : Chem., Biol. Clin. Aspects, 7th - pp. 299-303' 1983 , W. DE GRUYTER & CO. , BERLIN . NEW YORK<br>* page 301 *<br>--- | 1-12 | |
| A | CHEMICAL ABSTRACTS, vol. 92, no. 15, 14 April 1980, Columbus, Ohio, US;<br>abstract no. 123936s, J.J. MCCORMACK ET AL.<br>* abstract * & BIOCHEM. PHARMACOL. vol. 28, no. 21, 1979, pages 3227 - 9<br>--- -/- | 1-12 | |
| | | | TECHNICAL FIELDS SEARCHED (Int. Cl.5) |
| | | | C 07 D<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claims 13-15 are directed to a method of treatment of (diagnostic method practised on) the human/animal body (Art. 52(4) EPC) the search has been carried out and based on the alleged effects of the compound/ composition

| Place of search | Date of completion of the search | Examiner |
|---|---|---|
| BERLIN | 08-02-1993 | FRELON D L M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0407)

Page 2

European Patent Office

# PARTIAL EUROPEAN SEARCH REPORT

Application Number

EP 92 31 0232

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| A | CHEMICAL ABSTRACTS, vol. 115, no. 17, 28 October 1991, Columbus, Ohio, US; abstract no. 183227q, X.Y. MENG ET AL. * abstract * & YAOXUE XUEBAO vol. 26, no. 5, 1991, pages 383 - 6<br>----- | 1-12 | |
| | | | TECHNICAL FIELDS SEARCHED (Int. Cl.5) |

EPO FORM 1503 03.82 (P0410)